# EUROPEAN PATENT APPLICATION

(11) **EP 2 886 549 A1**
(43) Date of publication of application: **24.06.2015**
(21) Application number: 14196282.9
(22) Date of filing: 03.07.2006
(51) Int. Cl.: C07F 15/00, B01J 31/22, C07C 315/04, B01J 31/16, B01J 31/24, B01J 31/06, B01J 31/40

(54) **Ruthenium complex ligand, ruthenium complex and the use of the complex as a catalyst in olefin metathesis reactions**

(30) Priority: 04.07.2005 CN 200510080379
(62) Divisional of application: 06761350.5
(71) Applicant: Zannan Scitech Co., Ltd., Shanghai 201 108 (CN)
(72) Inventor: Zhan, Zheng-Yun, 201108 Shanghai (CN)
(74) Representative: Wohlfahrt, Jan Günther

(57) **Abstract**

The present invention disclosed a kind of ruthenium complex ligands, ruthenium complexes and immobilized ruthenium complex catalysts. The invention also relates to preparation and use of new ruthenium complex ligands and ruthenium complexes in metathesis reactions as catalysts have several significant advantages, e.g., high catalytic activity, high stability, easy recovery and reusability. The ruthenium complex ligand I and ruthenium complex II in the present invention have the following structures:

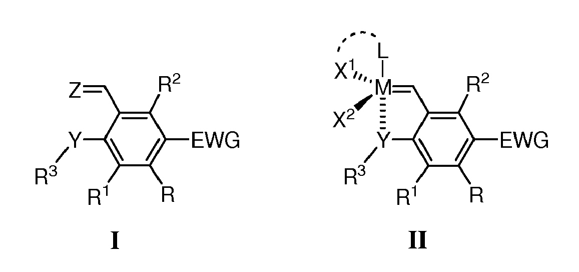

## Description

### FIELD OF THE INVENTION

The present invention relates to a kind of ruthenium (Ru) complex catalysts, especially relates to a kind of ruthenium complex ligands, ruthenium complexes, and immobilized ruthenium complex catalysts. The invention also relates to their preparation and use.

### BACKGROUND OF THE INVENTION

The study and development of ruthenium complex catalysts and its catalytic effect on olefin metathesis reactions led to popular attention in this field and broader application in the global organic chemical and pharmaceutical industries. Grubbs et al., inventors of ruthenium complex catalysts in this field, reported a variety of ruthenium complex catalysts whose catalytic activity and stability are limited to some extent. In order to enlarge the applying scope, these catalysts should be further optimized in the catalytic activity. For example, among the ruthenium complexes reported by Grubbs et al., RuCl₂(=CHPh)(PCy₃)₂ is comparatively effective in olefin metathesis reactions, but it is sensitive to air and water. It has disadvantages of lower catalytic activity and stability and decomposation at higher temperature.

Based on reported references of Ru complex catalysts, new nucleophilic complex ligands were used by Hoveyda et al. to improve the thermal stability and offered higher catalytic activity in metathesis reactions of multi-substituted olefins. However, the catalytic activity of these developed catalysts is still undesirable and there exists problems in separating the catalysts from the reaction mixture after the reaction is completed.

Therefore, disadvantages of Grubbs-Hoveyda catalysts are lower catalytic activity and stability, tendency to decompose at higher temperature. The catalytic activity in olefin metathesis reactions is lower for multi-substituted substrates.

### SUMMARY OF THE INVENTION

The present invention overcomes the above observed technical disadvantages by modifying substituents of Hoveyda complex ligands to obviously improve the catalytic activity of Ru complexes. In the present invention, the Ru complex ligands are characterized in that the substituents can be selected from electron-withdrawing substituent groups such as aminosulfonyl, sulfonamido and carbonyl. Because of introducing these substituents onto ruthenium complex ligands, the catalytic activity and stability of Ru complexes are significantly improved.

Therefore, in one aspect, the present invention provides a kind of ruthenium complex ligands having the following structure I: 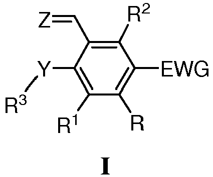 wherein:
Y is oxygen, sulfur, nitrogen or phosphorus;
Z is methylene, oxygen or p-toluenesulfonyl hydrazone;
R is hydrogen, halogen atom, nitro, cyano, C₁-C₂₀ alkyl, C₁-C₂₀ alkoxy, C₁-C₂₀ alkylthio, C₁-C₂₀ silanyl, C₁-C₂₀ silanyloxy, C₆-C₂₀ aryl, C₆-C₂₀ aryloxy, C₂-C₂₀ heterocyclic, C₂-C₂₀ heterocyclic aryl, sulfinyl, sulfonyl, formyl, C₁-C₂₀ carbonyl, C₁-C₂₀ ester, C₁-C₂₀ amido, C₁-C₂₀ uramido or derivatives or C₁-C₂₀ sulfonamido group;
R¹ and R² are each hydrogen, bromo (Br), iodo (I), C₁-C₂₀ alkyl or derivatives, C₁-C₂₀ alkoxy, C₁-C₂₀ alkylthio, C₁-C₂₀ silanyloxy, C₆-C₂₀ aryloxy, C₆-C₂₀ aryl, C₂-C₂₀ heterocyclic, C₂-C₂₀ heterocyclic aryl, C₁-C₂₀ ester , C₁-C₂₀ amido, C₁-C₂₀ uramido or derivatives or C₁-C₂₀ sulfonamido group;
R³ is hydrogen, C₁-C₂₀ alkyl or derivatives, C₁-C₂₀ alkoxy, C₁-C₂₀ alkylthio, C₁-C₂₀ silanyl, C₁-C₂₀ silanyloxy, C₆-C₂₀ aryl, C₆-C₂₀ aryloxy, C₂-C₂₀ heterocyclic, C₂-C₂₀ heterocyclic aryl, sulfinyl, sulfonyl, C₁-C₂₀ carbonyl, C₁-C₂₀ ester, C₁-C₂₀ amido, C₁-C₂₀ uramido or derivatives or C₁-C₂₀ sulfonamido group;
EWG is C₁-C₂₀ aminosulfonyl (R₂NSO₂), formyl, C₁-C₂₀ carbonyl, C₁-C₂₀ ester, C₁-C₂₀ aminocarbonyl (R₂NCO), amido, chloro, fluoro, C₁-C₂₀ uramido or derivatives or C₁-C₂₀ sulfonamido group.

In one preferred embodiment, the formula **I,**
wherein:
Y is oxygen or sulfur; Z is oxygen, methylene or p-toluenesulfonyl hydrazone;
R is hydrogen, halogen atom, nitro, cyano , C₁-C₁₅ alkyl, C₁-C₁₅ alkoxy, C₁-C₁₅ alkylthio, C₁-C₁₅ silanyl, C₁-C₁₅ silanyloxy, C₆-C₁₅ aryl, C₆-C₁₅ aryloxy, C₂-C₁₅ heterocyclic, C₂-C₁₅ heterocyclic aryl, sulfinyl, sulfonyl, formyl, C₁-C₁₅ carbonyl, C₁-C₁₅ ester, C₁-C₁₅ amido, C₁-C₁₅ uramido or derivatives or C₁-C₁₅ sulfonamido group;
R¹ and R² are each hydrogen , bromo (Br), iodo (I), C₁-C₁₅ alkyl or derivatives, C₁-C₁₅ alkoxy, C₁-C₁₅ alkylthio, C₁-C₁₅ silanyloxy, C₆-C₁₅ aryloxy, C₆-C₁₅ aryl, C₂-C₁₅ heterocyclic, C₂-C₁₅ heterocyclic aryl, C₁-C₁₅ ester, C₁-C₁₅ amido, C₁-C₁₅ uramido or derivatives or C₁-C₁₅ sulfonamido group;
R³ is hydrogen, C₁-C₁₅ alkyl or derivatives, C₁-C₁₅ alkoxy, C₁-C₁₅ alkylthio, C₁-C₁₅ silanyl, C₁-C₁₅ silanyloxy, C₆-C₁₅ aryl, C₆-C₁₅ aryloxy, C₂-C₁₅ heterocyclic, C₂-C₁₅ heterocyclic aryl, C₁-C₁₅ carbonyl, C₁-C₁₅ amido, C₁-C₁₅ uramido or derivatives or C₁-C₁₅ sulfonamido group;
EWG is C₁-C₁₅ aminosulfonyl (R₂NSO₂), formyl, C₁-C₁₅ carbonyl, C₁-C₁₅ ester, C₁-C₁₅ aminocarbonyl (R₂NCO), C₁-C₁₅ amido, chloro, fluoro, C₁-C₁₅ uramido or derivatives or C₁-C₁₅ sulfonamido group.

In another preferred embodiment, the formula **I,**
wherein:
Y is oxygen, Z is methylene or p-toluenesulfonyl hydrazone, R¹ and R² are each hydrogen, R³ is C₁-C₆ alkyl derivatives such as isopropyl or isobutyl ect., R is hydrogen, chloro, fluoro, C₁-C₈ carbonyl, C₁-C₈ ester, C₁-C₈ aminocarbonyl (R₂NCO), C₁-C₈ amido, C₁-C₈ uramido or derivatives or C₁-C₈ sulfonamido group;
EWG is an electron withdrawing group and selected from C₁-C₁₀ aminosulfonyl (R₂NSO₂), formyl, C₁-C₈ carbonyl, C₁-C₈ ester, C₁-C₈ aminocarbonyl (R₂NCO), C₁-C₈ amido, chloro, fluoro, C₁-C₈ uramido or derivatives or C₁-C₁₅ sulfonamido group.

In the second aspect, the present invention provides a kind of Ru complexes having the following structure **II:** 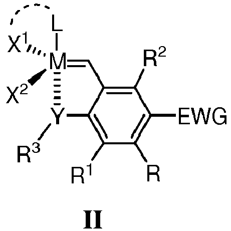 wherein:
M is ruthenium (Ru);
X¹ and X² are each chloro or RCOO, R is C₁-C₂₀ alkyl or derivatives;
L is an electron donating complex ligand, which could be linked or not linked with X¹ forming cyclic structure.
Y, R, R¹, R², R³, EWG each is as defined in the structure **I,** respectively.

In one preferred embodiment, L can be selected from following structures **IIIa, IIIb, IIIc** or **IIId:** 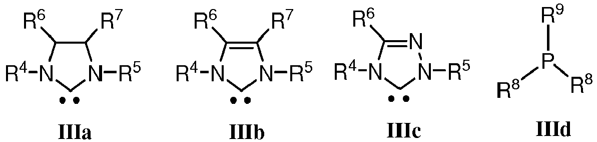 wherein:
R⁴ and R⁵ are each C₁-C₂₀ alkyl, C₆-C₂₀ aryl, C₂-C₂₀ heterocyclic aryl, C₁-C₂₀ heterocyclic, C₁-C₂₀ carbonyl, C₁-C₂₀ amido, C₁-C₂₀ uramido or derivatives or C₁-C₂₀ sulfonamido group;
R⁶ and R⁷ are each hydrogen, C₁-C₂₀ alkyl, C₁-C₂₀ alkoxy, C₁-C₂₀ alkylthio, C₁-C₂₀ silanyl, C₁-C₂₀ silanyloxy, C₆-C₂₀ aryl, C₆-C₂₀ aryloxy, C₂-C₂₀ heterocyclic aryl, C₂-C₂₀ heterocyclic, sulfinyl, sulfonyl, C₁-C₂₀ carbonyl, C₁-C₂₀ ester, C₁-C₂₀ amido, C₁-C₂₀ uramido or derivatives, C₁-C₂₀ sulfonamido, halogen atom, nitro or cyano group;
R⁸ and R⁹ are each C₁-C₂₀ alkyl or derivatives, C₁-C₂₀ alkoxy, C₆-C₂₀ aryl, C₆-C₂₀ aryloxy, C₂-C₂₀ heterocyclic aryl or C₂-C₂₀ heterocyclic group;
In another preferred embodiment, structural formula of L is **IIIa** or **IIId,** R⁴ and R⁵ are each 2,4,6-trimethylphenyl, R⁶ and R⁷ are each hydrogen or **IIId,** R⁸ and R⁹ are each cyclohexyl, respectively.

In one more preferred embodiment, the structure **II:**
wherein:
X¹ and X² are each chloro group;
L is **IIIa** or **IIIb;**
Y is oxygen;
R is hydrogen, halogen atom, nitro, cyano , C₁-C₁₅ alkyl, C₁-C₁₅ alkoxy, C₁-C₁₅ alkylthio, C₁-C₁₅ silanyl, C₁-C₁₅ silanyloxy, C₆-C₁₅ aryl, C₆-C₁₅ aryloxy, C₂-C₁₅ heterocyclic, C₂-C₁₅ heterocyclic aryl, sulfinyl, sulfonyl, formyl, C₁-C₁₅ carbonyl, C₁-C₁₅ ester, C₁-C₁₅ amido, C₁-C₁₅ uramido or derivatives or C₁-C₁₅ sulfonamido group;
R¹ and R² are each hydrogen , bromo (Br), iodo (I), C₁-C₁₅ alkyl or derivatives , C₁-C₁₅ alkoxy, C₁-C₁₅ alkylthio, C₁-C₁₅ silanyloxy, C₆-C₁₅ aryloxy, C₆-C₁₅ aryl, C₂-C₁₅ heterocyclic, C₂-C₁₅ heterocyclic aryl, C₁-C₁₅ ester , C₁-C₁₅ amido, C₁-C₁₅ uramido or derivatives or C₁-C₁₅ sulfonamido group;
R³ is hydrogen, C₁-C₁₅ alkyl or derivatives, C₁-C₁₅ alkoxy, C₁-C₁₅ alkylthio, C₁-C₁₅ silanyl, C₁-C₁₅ silanyloxy, C₆-C₁₂ aryl, C₆-C₁₂ aryloxy, C₂-C₁₂ heterocyclic, C₂-C₁₂ heterocyclic aryl, C₁-C₁₂ carbonyl, C₁-C₁₂ amido, C₁-C₁₂ uramido or derivatives or C₁-C₁₂ sulfonamido group;
EWG is C₁-C₁₅ amidosulfonyl (R₂NSO₂), formyl, C₁-C₁₅ carbonyl, C₁-C₁₅ ester, C₁-C₁₅ aminocarbonyl (R₂NCO), C₁-C₁₅ amido, chloro, fluoro, C₁-C₁₅ uramido or derivatives or C₁-C₁₅ sulfonamido group.

In one preferred embodiment, the structure **IIIa:**
R⁴ and R⁵ are each aryl group; R⁶ and R⁷ are each hydrogen, respectively.

In the best embodiment, the structure **II:**
R¹ and R² are each hydrogen, R³ is C₁-C₆ alkyl derivatives such as isopropyl and isobutyl, R is hydrogen, chloro, fluoro, C₁-C₈ carbonyl, C₁-C₈ ester, C₁-C₈ aminocarbonyl (R₂NCO), C₁-C₈ amido, C₁-C₈ uramido or derivatives or C₁-C₈ sulfonamido group; EWG is an electron withdrawing group selected from C₁-C₁₀ amidosulfonyl (R₂NSO₂), formyl, C₁-C₈ carbonyl, C₁-C₈ ester, C₁-C₈ aminocarbonyl (R₂NCO), C₁-C₈ amido, chloro, fluoro, C₁-C₈ uramido or derivatives or C₁-C₁₅ sulfonamido group.

In the present invention, based on different surface functional groups "X", ruthenium complex catalysts could be chemically bounded to the surface of macromolecular materials such as resins, polyethylene glycol(PEG), poly styrene, silica gel to form the corresponding products of immobilized ruthenium complex catalysts, which are reusable and beneficial to the work-up and purification after metathesis reactions, and also easily recycable to avoid environmental pollution.

Then, in the third aspect, the present invention also provides an immobilized ruthenium complex catalysts having the following structure **IVa-IVd:** 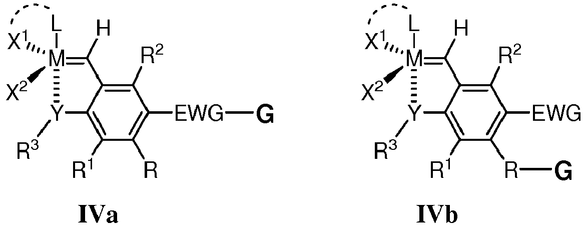 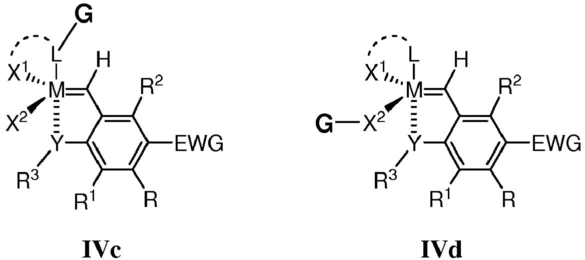 wherein,
G is a kind of macromolecular materials selected from polymers, resins, PEGs, or silica gels having functional group "X³" on the surface. Functional group "X³" on the surface is hydroxyl, amino, thiol, carboxyl, C₁-C₂₀ alkyl or derivatives, C₁-C₂₀ alkoxy, C₁-C₂₀ alkylthio, C₁-C₂₀ silanyl, C₁-C₂₀ silanyloxy, C₆-C₂₀ aryloxy, C₂-C₂₀ heterocyclic, sulfinyl, sulfonyl, C₁-C₂₀ carbonyl, C₁-C₂₀ ester, amino, C₁-C₂₀ amido, C₁-C₂₀ uramido or derivatives or C₁-C₂₀ sulfonamido group;
X¹, X², R, R¹, R², R³, Y, L, EWG, M each is as defined above respectively.

Wherein polymers in structural formulas above can be resins with hydroxyl group on the surface (1.2-2.0 mmol/g, Tianjing Nankai Synthesis Science and Technological Company Limited), silica gel and PEG (molecular weight of 200-4000, Shanghai Chemical Reagents Company) and so on. Polystyrene polymer or cross-linked polystyrene resins are preferred. Cross-linked polystyrene resin that is preferably used in the present invention as immobilized material is connected to the aminosulfonyl-substituted ligands on the surface of immobilized material through coupling reaction, then reacts with Ru complex **1** to form new immobilized Ru complex catalysts **18** and **19** by complexation reaction. Polystyrene polymer-immobilized Ru catalysts such as catalysts **18** and **19** as a kind of resin-immobilized catalysts are useful in the olefin metathesis reactions, and can be recovered by precipitation and filtration from solvents such as methanol etc., and reused.

In a preferred embodiment, **L** in the structure **IV** of immobilized Ru complex catalysts is selected from formula **IIIa, IIIb, IIIc** or **IIId:** 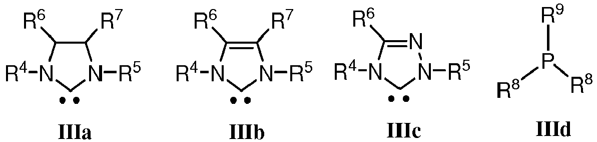 Wherein:
R⁴ and R⁵ are each C₁-C₂₀ alkyl, C₆-C₂₀ aryl, C₂-C₂₀ heterocyclic aryl, C₁-C₂₀ heterocyclic, C₁-C₂₀ carbonyl, C₁-C₂₀ amido, C₁-C₂₀ uramido or derivatives or C₁-C₂₀ sulfonamido group;
R⁶ and R⁷ are each hydrogen, C₁-C₂₀ alkyl, C₁-C₂₀ alkoxy, C₁-C₂₀ alkylthio, C₁-C₂₀ silanyl, C₁-C₂₀ silanyloxy, C₆-C₂₀ aryl, C₆-C₂₀ aryloxy, C₂-C₂₀ heterocyclic aryl, C₂-C₂₀ heterocyclic, sulfinyl, sulfonyl, C₁-C₂₀ carbonyl, C₁-C₂₀ ester , C₁-C₂₀ amido, C₁-C₂₀ uramido or derivatives, C₁-C₂₀ sulfonamido, halogen atom, nitro or cyano group;
R⁸ and R⁹ are each C₁-C₂₀ alkyl or derivatives, C₁-C₂₀ alkoxy, C₆-C₂₀ aryl, C₆-C₂₀ aryloxy, C₂-C₂₀ heterocyclic aryl or C₂-C₂₀ heterocyclic group;

In a preferred embodiment, the structural formula of **L** is formula **IIIa,** R⁴ and R⁵ are each aryl, R⁶ and R⁷ are each hydrogen or formula **IIIb,** R⁸ and R⁹ are each cyclohexyl, respectively.

In the best embodiment, formula **IVa,** where X is oxygen; **G** is polystyrene resin with hydroxyl group on the surface or polyethylene glycol; **Y** is oxygen; R¹ and R² are each hydrogen; R³ is isopropyl or C₁-C₆ alkyl group; R is hydrogen, chloro, fluoro, C₁-C₈ carbonyl, C₁-C₈ ester, C₁-C₈ aminocarbonyl (R₂NCO), C₁-C₈ amido, C₁-C₈ uramido or derivatives or C₁-C₈ sulfonamido group; EWG is an electron withdrawing group selected from C₁-C₁₀ aminosulfonyl (R₂NSO₂), formyl, C₁-C₈ carbonyl, C₁-C₈ ester, C₁-C₈ aminocarbonyl (R₂NCO), C₁-C₈ amido, chloro, fluoro, C₁-C₈ uramido or derivatives or C₁-C₁₅ sulfonamido group; R⁴ and R⁵ are each 2,4,6- trimethylphenyl group.

In the fourth aspect, the present invention provides a method of preparation of ruthenium complex catalysts which includes these following steps:
(1) Under the protection of inert gas, p-toluenesulfonyl hydrazone and NaOEt or NaOMe are added together in ethanol to produce ortho-alkoxycarbene intermediate, then react with RuCl₂P(Ph₃)₃ to produce the following Ru complex **V:** 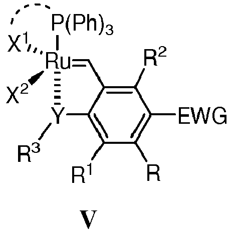 wherein:
   X¹, X², Y, R, R¹, R², R³ and EWG each is as defined as claims 4-9 respectively.
(2) Under the protection of inert gas, Ru complex product **V** reacts with tricyclohexyl phosphine (PCy₃) to produce the following Ru complex **VI:** 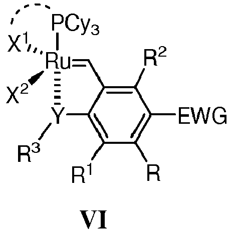 wherein:
   X¹, X², Y, R, R¹, R², R³ and EWG each is as defined as claims 4-9 respectively.
(3) According to the chemical activity, under the protection of inert gas, the complex of formula **V** of step 1 or complex **VI** of step 2 is used to prepare Ru complex catalyst **II** of claim 4.

In the fifth aspect, the present invention provides a method of preparation of immobilized ruthenium complex catalysts, and the preparation steps are as follows:
(1) Vinylation product of ester-substituted 4-hydroxybenzensulfonamide is obtained by vinylation at ortho-position of hydroxy of 4-hydroxybenzensulfonamide;
(2) Etherification of the above vinylated ortho-phenol product (step 1) with alkyl halide offers the etherified product;
(3) Hydrolysis of the etherified product from step 2 in basic solution offers hydrolytic product;
(4) In the presence of coupling reagent, hydrolytic product of step 3 reacts with either hydroxyl or amino group on the surface of macromolecular substance to gain immobilized ruthenium ligand;
(5) Immobilized ligand of step 4 reacts with RuCl₂ (=CHPh)(PPh₃)₂ to gain Ru intermediate product;
(6) The final product is produced by the reaction of immobilized Ru complex intermediate of step 5 with tricyclohexyl phosphine (PCy₃) ligand or another ligand H₂IMes (**IIIa**).

In a preferred embodiment, the vinylation of step 1: under the protection of inert gas, tertiary amine (twice to thrice by volume, i.e. 2-3 BV) is added to tin tetrachloride (SnCl₄, 1 BV) and 1,2-dichloroethane (DCE, 3-6 BV) solution at -30 °C to -50°C with inlet of acetylene for 4∼6 hours, and ester-substituted 4-hydroxybenzensulfonamide is added for vinylation at room temperature, then vinylated ortho-phenol product is produced at 60∼100°C;
Step 2: the vinylated ortho-phenol product of step1 reacts with alkyl halide to offer etherification product;
Step 5: the immobilized Ru ligand of step 4 reacts with RuCl₂ (=CHPh)(H₂IMes) and CuCl in solvent, e.g., DCM, DCE, toluene, etc.

In a preferred embodiment, the inert gas of step 1 is argon, tertiary amine (2 BV) is added to tin tetrachloride (1 BV) and 1,2-dichloroethane (3.5 BV) solution at -40°C with inlet of acetylene for 6 hours, then ester-substituted 4-hydroxybenzensulfonamide is added at room temperature to produce vinylated ortho-phenol product at 80°C;
Step 2: the vinylated ortho-phenol product of step1 reacts with isopropyl iodide in dimethyl formamide solution by etherification;
Step 3: hydrolysis is carried out in NaOH-alcohol or NaOH -H₂O solution;
Step 4: in the presence of dicyclohexylcarbodiimide (DCC), the hydrolytic product of step 3 reacts with either hydroxyl or amino group on the surface of polystyrene resin to gain the immobilized Ru ligand;
Step 5: the immobilized Ru complex ligand of step 4 reacts with RuCl₂ (=CHPh)(PPh₃) and CuCl in dichloromethane (DCM) to gain immobilized ruthenium complex.
Step 6: The final product is gained by the reaction of immobilized Ru complex of the step 5 with tricyclohexyl phosphine (PCy₃) ligand or another ligand H₂IMes (**IIIa**) in dichloromethane.

The ruthenium complex ligands and ruthenium complexes in the present invention can be prepared based on four alternative procedures in the following Schemes 1-4, respectively:

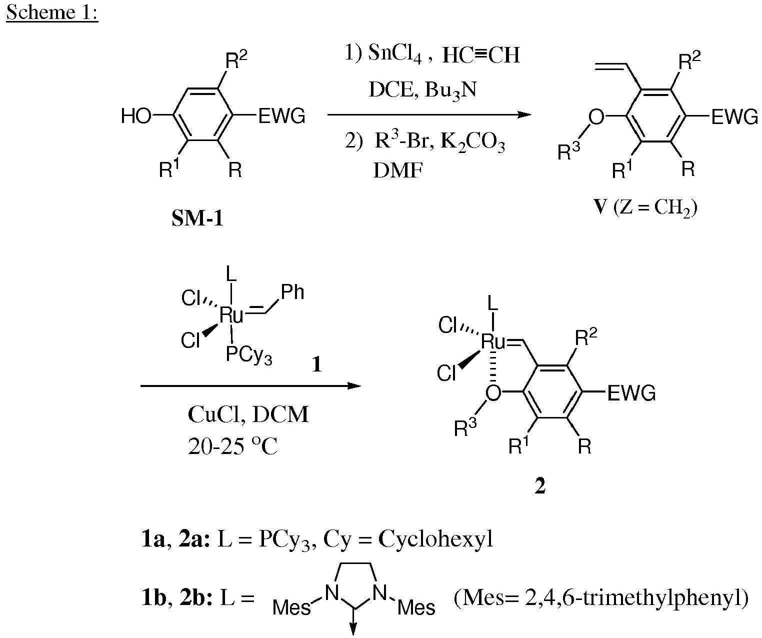

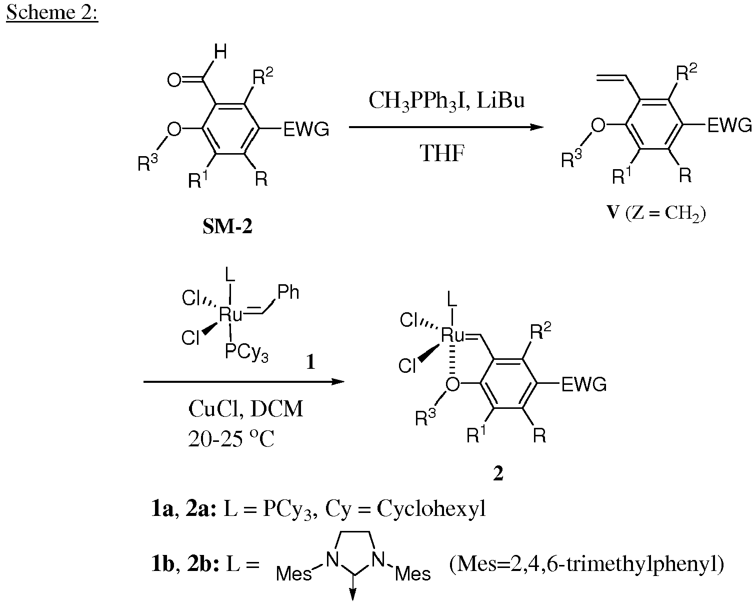

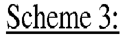

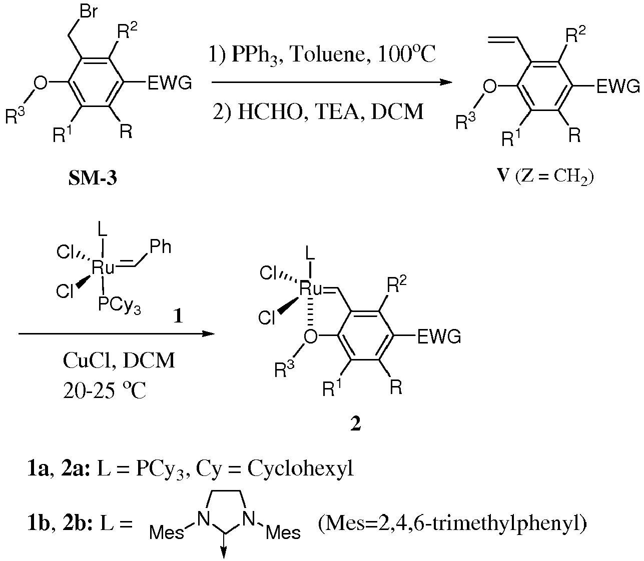

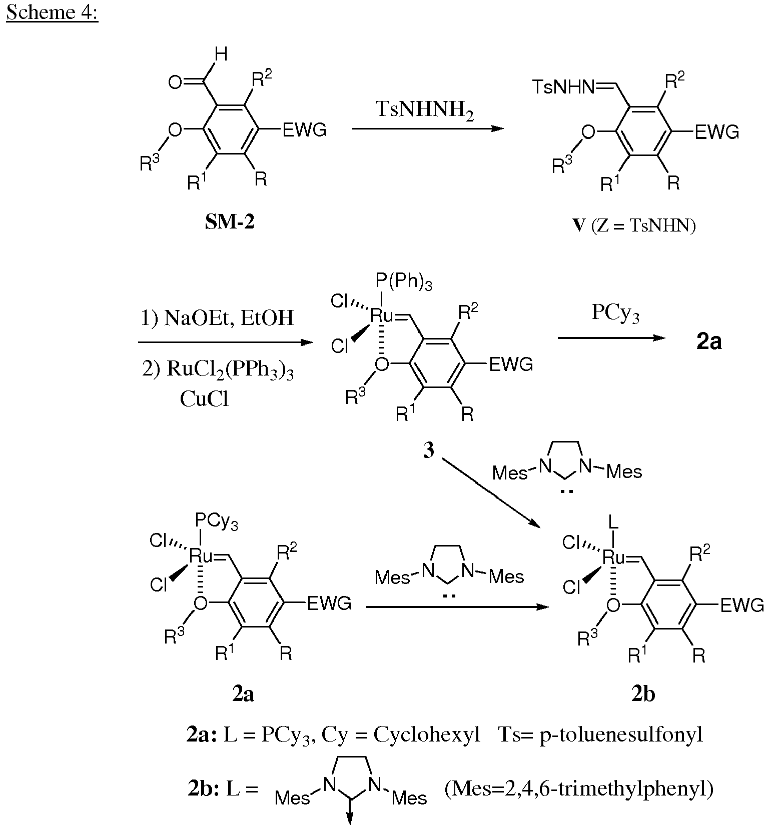

In the above schemes, R, R¹, R², R³ and EWG in structural formulas SM-1, SM-2 and SM-3 each is as defined above respectively.

When Z is methylene (CH₂), it is convenient to carry out the preparation by the procedures in schemes 1-3. The Ru complex ligands and complexes produced from electron-withdrawing group (aminosulfonyl, sulfonamide, etc.) substituted ortho-alkoxystyrene are prepared by electron-withdrawing group (aminosulfonyl, sulfonamide, carbonyl, etc.) substituted phenol. In Scheme 1, it was reported by M. Yamaguchi et al. (J. Org. Chem. 1998, 63, 7298-7305); in Scheme 2, it was reported by Hoveyda et al. (J. Am. Chem. Soc. 1999, 121, 791-799).

When Z is oxygen, according to Scheme 4, by the reaction of electron withdrawing group (aminosulfonyl, sulfonamide, etc.) substituted ortho-alkoxyphenyl aldehyde and p-toluenesulfonyl hydrazine leads to p-toluenesulfonyl hydrazone, under the protection of inert gas, which with sodium ethoxide (NaOEt) or sodium methoxide (NaOMe) in ethanol produces ortho-alkoxycarbene. And ortho-alkoxycarbene reacts with RuCl₂P(Ph₃)₃ to produce Ru complex **(V)** with triphenylphosphine. Under the protection of inert gas, Ru complex **VI** is obtained by incorporating complex **V** with tricyclohexyl phosphine. According to the chemical activity, under the protection of inert gas, the complex **V** or **VI** reacts with five-membered heterocyclic ligand **II** to produce Ru complex catalyst II.

In the sixth aspect, the present invention provides the use of Ru complex **II** as catalyst in olefin metathesis reactions.

Wherein, the described olefin metathesis reactions include intramolecular ring-closing olefin metathesis reactions, intermolecular olefin metathesis reactions, and olefin metathesis polymerization reactions.

In the seventh aspect, the present invention provides the catalytic use of immobilized Ru complex **IV** as recycable catalyst in olefin metathesis reactions.

Wherein, the described olefin metathesis reactions include intramolecular ring-closing olefin metathesis reactions, intermolecular olefin metathesis reactions, and olefin metathesis polymerization reactions.

The present invention provides the following significant achievements:
1. In the invention, the ruthenium complex ligands and corresponding ruthenium complexes have been designed and synthesized, and the ligand effect of substituents at different phenyl position on the catalytic activity and stability of Ru catalysts. The results show that ortho-alkoxystyrene complex ligands with the electron-withdrawing substituted group such as aminosulfonyl, sulfonamido, carbonyl and chloro group remarkably promote the catalytic activity and stability of corresponding Ru complex catalysts. As highly-active catalysts, they can be used efficiently in different kinds of olefin metathesis reactions, e.g., intramolecular ring-closing olefin metathesis reactions, intermolecular olefin metathesis reactions and polymerization reactions etc., and have a broad range of application value in industries. These new and highly-active metathesis catalysts provide a useful alternative method in production of new chemical materials and drug R&D.
2. In the invention, Ru complex ligands are linked on the surface of polymers such as resins, polyethylene glycol, polystyrene and silica gel with hydroxyl or amino group on the surface, and followed by reacting with Ru complexes to generate new complex catalysts, i.e. immobilized Ru complex catalysts. The advantage of these new immobilized Ru catalysts is easily recycable and reusable in metathesis, which is a kind of valuably developed metathesis catalysts in "Green Chemistry".
3. Based on study of the substituent effect on catalytic activity with a diversity of ligands, the invention provides the new Ru catalysts which have much better catalytic activity than Grubbs-Hoveyda catalysts, and also provides new improved methods well-developed for preparation of different Ru catalysts which obviously reduces the production costs. Finally, the present invention provides an effective and practical method to carry out the olefin metathesis reactions with some new highly active Ru catalysts in industry.

Furthermore, the present invention relates to the following aspects:
Aspect 1: A ruthenium complex ligand having the following structure **I:** 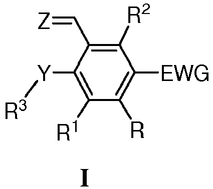 wherein:
   Y is oxygen, sulfur, nitrogen or phosphorus;
   Z is methylene, oxygen or p-toluenesulfonyl hydrazone;
   R is hydrogen, halogen atom, nitro, cyano, C₁-C₂₀ alkyl, C₁-C₂₀ alkoxy, C₁-C₂₀ alkylthio, C₁-C₂₀ silanyl, C₁-C₂₀ silanyloxy, C₆-C₂₀ aryl, C₆-C₂₀ aryloxy, C₂-C₂₀ heterocyclic, C₂-C₂₀ heterocyclic aryl, sulfinyl, sulfonyl, formyl, C₁-C₂₀ carbonyl, C₁-C₂₀ ester, C₁-C₂₀ amido, C₁-C₂₀ uramido or derivatives, or C₁-C₂₀ sulfonamido group;
   R¹ and R² are each hydrogen , bromo (Br), I, C₁-C₂₀ alkyl or derivatives , C₁-C₂₀ alkoxy, C₁-C₂₀ alkylthio, C₁-C₂₀ silanyloxy, C₆-C₂₀ aryloxy, C₆-C₂₀ aryl, C₂-C₂₀ heterocyclic, C₂-C₂₀ heterocyclic aryl, C₁-C₂₀ ester, C₁-C₂₀ amido, C₁-C₂₀ uramido or derivatives, or C₁-C₂₀ sulfonamido group;
   R³ is hydrogen, C₁-C₂₀ alkyl or derivatives , C₁-C₂₀ alkoxy, C₁-C₂₀ alkylthio, C₁-C₂₀ silanyl, C₁-C₂₀ silanyloxy, C₆-C₂₀ aryl, C₆-C₂₀ aryloxy, C₂-C₂₀ heterocyclic, C₂-C₂₀ heterocyclic aryl, sulfinyl, sulfonyl, C₁-C₂₀ carbonyl, C₁-C₂₀ ester, C₁-C₂₀ amido, C₁-C₂₀ uramido or derivatives, or C₁-C₂₀ sulfonamido group;
   EWG is C₁-C₂₀ aminosulfonyl (R₂NSO₂), formyl, C₁-C₂₀ carbonyl, C₁-C₂₀ ester, C₁-C₂₀ aminocarbonyl (R₂NCO), amido, chloro, fluoro, C₁-C₂₀ uramido or derivatives, or C₁-C₂₀ sulfonamido group.

Aspect 2: The ruthenium complex ligand according to aspect 1, wherein in structure **I,**
Y is oxygen or sulfur;
Z is oxygen, methylene or p-toluenesulfonyl hydrazone;
R is hydrogen, halogen atom, nitro, cyano , C₁-C₁₅ alkyl, C₁-C₁₅ alkoxy, C₁-C₁₅ alkylthio, C₁-C₁₅ silanyl, C₁-C₁₅ silanyloxy, C₆-C₁₅ aryl, C₆-C₁₅ aryloxy, C₂-C₁₅ heterocyclic, C₂-C₁₅ heterocyclic aryl, sulfinyl, sulfonyl, formyl, C₁-C₁₅ carbonyl, C₁-C₁₅ ester, C₁-C₁₅ amido, C₁-C₁₅ uramido or derivatives, or C₁-C₁₅ sulfonamido group;
R¹ and R² are each hydrogen, Bromo (Br), I, C₁-C₁₅ alkyl or derivatives , C₁-C₁₅ alkoxy, C₁-C₁₅ alkylthio, C₁-C₁₅ silanyloxy, C₆-C₁₅ aryloxy, C₆-C₁₅ aryl, C₂-C₁₅ heterocyclic, C₂-C₁₅ heterocyclic aryl, C₁-C₁₅ ester, C₁-C₁₅ amido, C₁-C₁₅ uramido or derivatives, or C₁-C₁₅ sulfonamido group;
R³ is hydrogen, C₁-C₁₅ alkyl or derivatives, C₁-C₁₅ alkoxy, C₁-C₁₅ alkylthio, C₁-C₁₅ silanyl, C₁-C₁₅ silanyloxy, C₆-C₁₅ aryl, C₆-C₁₅ aryloxy, C₂-C₁₅ heterocyclic, C₂-C₁₅ heterocyclic aryl, C₁-C₁₅ carbonyl, C₁-C₁₅ amido, C₁-C₁₅ uramido or derivatives, or C₁-C₁₅ sulfonamido group;
EWG is C₁-C₁₅ aminosulfonyl (R₂NSO₂), formyl, C₁-C₁₅ carbonyl, C₁-C₁₅ ester, C₁-C₁₅ aminocarbonyl (R₂NCO), C₁-C₁₅ amido, chloro, fluoro, C₁-C₁₅ uramido or derivatives, or C₁-C₁₅ sulfonamido group.

Aspect 3: The ruthenium complex ligand according to aspect 2, wherein in structure I, Y is oxygen, Z is methylene or p-toluenesulfonyl hydrazone, R¹ and R² are each hydrogen, R³ is C₁-C₆ alkyl derivatives such as isopropyl or isobutyl ect., R is hydrogen, chloro fluoro, C₁-C₈ carbonyl, C₁-C₈ ester, C₁-C₈ aminocarbonyl (R₂NCO), C₁-C₈ amido, C₁-C₈ uramido or derivatives, or C₁-C₈ sulfonamido group; EWG is an electron withdrawing group selected from C₁-C₁₀ aminosulfonyl (R₂NSO₂), formyl, C₁-C₈ carbonyl, C₁-C₈ ester, C₁-C₈ aminocarbonyl (R₂NCO), C₁-C₈ amido, chloro, fluoro, C₁-C₈ uramido or derivatives, or C₁-C₁₅ sulfonamido group.

Aspect 4: A ruthenium complex having the following structure **II:** 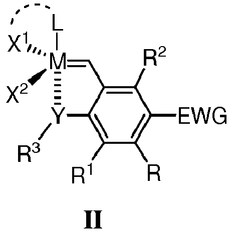 wherein,
M is ruthenium (Ru);
X¹ and X² are each chloro or RCOO, R is C₁-C₂₀ alkyl or derivatives;
L is an electron donating complex ligand, which could be linked or not linked with X¹ forming cyclic structure.
Y, R, R¹, R², R³ and EWG each is as defined in aspect 1, respectively.

Aspect 5: The ruthenium complex according to aspect 4, wherein L can be selected from the following structures **IIIa, IIIb, IIIc** or **IIId:** 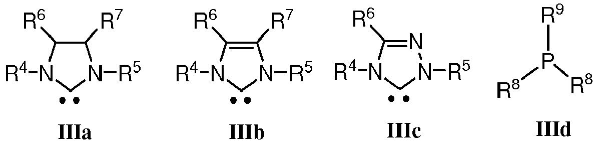 wherein:
R⁴ and R⁵ are each C₁-C₂₀ alkyl, C₆-C₂₀ aryl, C₂-C₂₀ heterocyclic aryl, C1-C₂₀ heterocyclic, C₁-C₂₀ carbonyl, C₁-C₂₀ amido, C₁-C₂₀ uramido or derivatives, or C₁-C₂₀ sulfonamido group;
R⁶ and R⁷ are each hydrogen, C₁-C₂₀ alkyl, C₁-C₂₀ alkoxy, C₁-C₂₀ alkylthio, C₁-C₂₀ silanyl, C₁-C₂₀ silanyloxy, C₆-C₂₀ aryl, C₆-C₂₀ aryloxy, C₂-C₂₀ heterocyclic aryl, C₂-C₂₀ heterocyclic, sulfinyl, sulfonyl, C₁-C₂₀ carbonyl, C₁-C₂₀ ester, C₁-C₂₀ amido, C₁-C₂₀ uramido or derivatives, C₁-C₂₀ sulfonamido, halogen atom, nitro or cyano group;
R⁸ and R⁹ are each C₁-C₂₀ alkyl or derivatives, C₁-C₂₀ alkoxy, C₆-C₂₀ aryl, C₆-C₂₀ aryloxy, C₂-C₂₀ heterocyclic aryl, or C₂-C₂₀ heterocyclic group;

Aspect 6: The ruthenium complex according to aspect 5, wherein structural formula of L is structure **IIIa,** R⁴ and R⁵ are each 2,4,6-trimethylphenyl, R⁶ and R⁷ are each hydrogen or **IIId,** R⁸ and R⁹ are each cyclohexyl.

Aspect 7: The ruthenium complex according to aspect 5, wherein in structure **II,**
X¹ and X² are each chloro anion;
L is **IIIa** or **IIId;**
Y is oxygen;
R is hydrogen, halogen atom, nitro, cyano , C₁-C₁₅ alkyl, C₁-C₁₅ alkoxy, C₁-C₁₅ alkylthio, C₁-C₁₅ silanyl, C₁-C₁₅ silanyloxy, C₆-C₁₅ aryl, C₆-C₁₅ aryloxy, C₂-C₁₅ heterocyclic, C₂-C₁₅ heterocyclic aryl, sulfinyl, sulfonyl, formyl, C₁-C₁₅ carbonyl, C₁-C₁₅ ester, C₁-C₁₅ amido, C₁-C₁₅ uramido or derivatives, or C₁-C₁₅ sulfonamido group;
R¹ and R² are each hydrogen, bromo (Br), iodo (I), C₁-C₁₅ alkyl or derivatives, C₁-C₁₅ alkoxy, C₁-C₁₅ alkylthio, C₁-C₁₅ silanyloxy, C₆-C₁₅ aryloxy, C₆-C₁₅ aryl, C₂-C₁₅ heterocyclic, C₂-C₁₅ heterocyclic aryl, C₁-C₁₅ ester, C₁-C₁₅ amido, C₁-C₁₅ uramido or derivatives, or C₁-C₁₅ sulfonamido group;
R³ is hydrogen, C₁-C₁₅ alkyl or derivatives, C₁-C₁₅ alkoxy, C₁-C₁₅ alkylthio, C₁-C₁₅ silanyl, C₁-C₁₅ silanyloxy, C₆-C₁₂ aryl, C₆-C₁₂ aryloxy, C₂-C₁₂ heterocyclic, C₂-C₁₂ heterocyclic aryl, C₁-C₁₂ carbonyl, C₁-C₁₂ amido, C₁-C₁₂ uramido or derivatives, or C₁-C₁₂ sulfonamido group;
EWG is C₁-C₁₅ aminosulfonyl (R₂NSO₂), formyl, C₁-C₁₅ carbonyl, C₁-C₁₅ ester, C₁-C₁₅ aminocarbonyl (R₂NCO), C₁-C₁₅ amido, chloro, fluoro, C₁-C₁₅ uramido or derivatives, or C₁-C₁₅ sulfonamido group.

Aspect 8: The ruthenium complex according to aspect 7, wherein in **IIIa,** R⁴ and R⁵ are each aryl group, R⁶ and R⁷ are each hydrogen.

Aspect 9: The ruthenium complex according to aspect 8, wherein in the structure **II**, Y is oxygen, R¹ and R² are each hydrogen, R³ is isopropyl or C₁-C₆ alkyl, R is hydrogen, chloro, fluoro, C₁-C₈ carbonyl, C₁-C₈ ester, C₁-C₈ aminocarbonyl (R₂NCO), C₁-C₈ amido, C₁-C₈ uramido or derivatives or C₁-C₈ sulfonamido group; EWG is an electron withdrawing group and selected from C₁-C₁₀ aminosulfonyl (R₂NSO₂), formyl, C₁-C₈ carbonyl, C₁-C₈ ester, C₁-C₈ aminocarbonyl (R₂NCO), C₁-C₈ amido, chloro, fluoro, C₁-C₈ uramido or derivatives, or C₁-C₁₅ sulfonamido, R⁴ and R⁵ are each 2,4,6-trimethylphenyl group, respectively.

Aspect 10: An immobilized ruthenium complex catalyst having the following structure **IVa, IVb, IVc, IVd:** 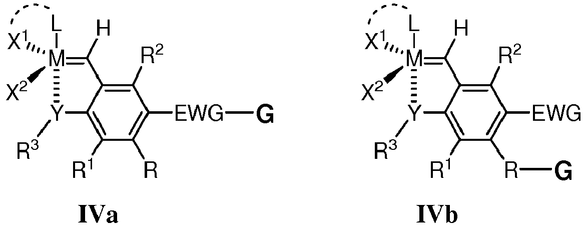 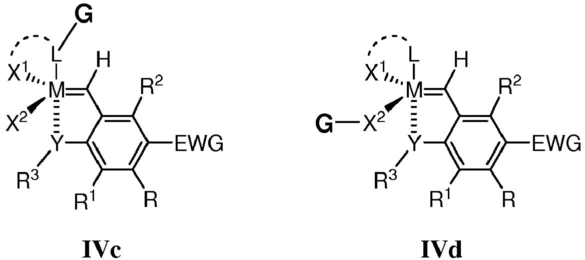 wherein,
G is a kind of macromolecular materials selected from polymers, resins, polyethylene glycol (PEG), or silica gels having functional group "X³" on the surface. Functional group "X³" is hydroxyl, amino, thiol, carboxyl, C₁-C₂₀ alkyl or derivatives, C₁-C₂₀ alkoxy, C₁-C₂₀ alkylthio, C₁-C₂₀ silanyl, C₁-C₂₀ silanyloxy, C₆-C₂₀ aryloxy, C₂-C₂₀ heterocyclic, sulfinyl, sulfonyl, C₁-C₂₀ carbonyl, C₁-C₂₀ ester, amino, C₁-C₂₀ amido, C₁-C₂₀ uramido or derivatives or C₁-C₂₀ sulfonamido group;
M, L, X¹, X², Y, R, R¹, R², R³ and EWG each is as defined in aspect 4.

Aspect 11: The immobilized ruthenium complex catalyst according to aspect 10, wherein structural formula of L can be selected from following structures **IIIa, IIIb, IIIc** or **IIId:** 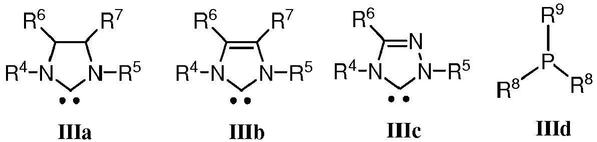 wherein:
R⁴ and R⁵ are each C₁-C₂₀ alkyl, C₆-C₂₀ aryl, C₂-C₂₀ heterocyclic aryl, C₁-C₂₀ heterocyclic, C₁-C₂₀ carbonyl, C₁-C₂₀ amido, C₁-C₂₀ uramido or derivatives, or C₁-C₂₀ sulfonamido group;
R⁶ and R⁷ are each hydrogen, C₁-C₂₀ alkyl, C₁-C₂₀ alkoxy, C₁-C₂₀ alkylthio, C₁-C₂₀ silanyl, C₁-C₂₀ silanyloxy, C₆-C₂₀ aryl, C₆-C₂₀ aryloxy, C₂-C₂₀ heterocyclic aryl, C₂-C₂₀ heterocyclic, sulfinyl, sulfonyl, C₁-C₂₀ carbonyl, C₁-C₂₀ ester, C₁-C₂₀ amido, C₁-C₂₀ uramido or derivatives, C₁-C₂₀ sulfonamido, halogen atom, nitro or cyano group;
R⁸ and R⁹ are each C₁-C₂₀ alkyl or derivatives, C₁-C₂₀ alkoxy, C₆-C₂₀ aryl, C₆-C₂₀ aryloxy, C₂-C₂₀ heterocyclic aryl, or C₂-C₂₀ heterocyclic group;

Aspect 12: The immobilized ruthenium complex catalyst according to aspect 11, wherein structural formula of L is structure **IIIa,** R⁴ and R⁵ are each aryl, R⁶ and R⁷ are each hydrogen or **IIId,** R⁸ and R⁹ are each cyclohexyl.

Aspect 13: The immobilized ruthenium complex catalyst according to aspect 11, wherein in structure **IVa, IVb, IVc, IVd,**
Y is oxygen;
X¹ and X² are each chloro anion;
L is **IIIa** or **IIId;**
G is resins with hydroxyl, amino, thiol or carboxyl group on the surface, rubbers, silica gel or PEGs;
R is hydrogen, halogen atom, nitro, cyano, C₁-C₁₅ alkyl, C₁-C₁₅ alkoxy, C₁-C₁₅ alkylthio, C₁-C₁₅ silanyl, C₁-C₁₅ silanyloxy, C₆-C₁₅ aryl, C₆-C₁₅ aryloxy, C₂-C₁₅ heterocyclic, C₂-C₁₅ heterocyclic aryl, sulfinyl, sulfonyl, formyl, C₁-C₁₅ carbonyl, C₁-C₁₅ ester, C₁-C₁₅ amido, C₁-C₁₅ uramido or derivatives, or C₁-C₁₅ sulfonamido group;
R¹ and R² are each hydrogen, bromo (Br), iodo (I), C₁-C₁₅ alkyl or derivatives, C₁-C₁₅ alkoxy, C₁-C₁₅ alkylthio, C₁-C₁₅ silanyloxy, C₆-C₁₅ aryloxy, C₆-C₁₅ aryl, C₂-C₁₅ heterocyclic, C₂-C₁₅ heterocyclic aryl, C₁-C₁₅ ester, C₁-C₁₅ amido, C₁-C₁₅ uramido or derivatives, or C₁-C₁₅ sulfonamido group;
R³ is hydrogen, C₁-C₁₅ alkyl or derivatives, C₁-C₁₅ alkoxy, C₁-C₁₅ alkylthio, C₁-C₁₅ silanyl, C₁-C₁₅ silanyloxy, C₆-C₁₂ aryl, C₆-C₁₂ aryloxy, C₂-C₁₂ heterocyclic, C₂-C₁₂ heterocyclic aryl, C₁-C₁₂ carbonyl, C₁-C₁₂ amido, C₁-C₁₂ uramido or derivatives, or C₁-C₁₂ sulfonamido group;
EWG is C₁-C₁₅ aminosulfonyl (R₂NSO₂), formyl, C₁-C₁₅ carbonyl, C₁-C₁₅ ester, C₁-C₁₅ aminocarbonyl (R₂NCO), C₁-C₁₅ amido, chloro, fluoro, C₁-C₁₅ uramido or derivatives, or C₁-C₁₅ sulfonamido group.

Aspect 14: The immobilized ruthenium complex catalyst according to aspect 13, wherein in structure **IIIa,** R⁴ and R⁵ are each aryl, R⁶ and R⁷ are each hydrogen or structure **IIId,** R⁸ and R⁹ are each cyclohexyl.

Aspect 15: The immobilized ruthenium complex catalyst according to aspect 14, wherein in structure **IVa,** G is polystyrene resin with hydroxyl group on the surface or PEG, Y is oxygen, R¹ and R² are each hydrogen, R³ is isopropyl or C₁-C₆ alkyl, R is hydrogen, chloro fluoro, C₁-C₈ carbonyl, C₁-C₈ ester, C₁-C₈ aminocarbonyl (R₂NCO), C₁-C₈ amido, C₁-C₈ uramido or derivatives, or C₁-C₈ sulfonamido group; EWG is an electron withdrawing group and selected from C₁-C₁₀ aminosulfonyl (R₂NSO₂), formyl, C₁-C₈ carbonyl, C₁-C₈ ester, C₁-C₈ aminocarbonyl (R₂NCO), C₁-C₈ amido, chloro, fluoro, C₁-C₈ uramido or derivatives, or C₁-C₁₅ sulfonamido, R⁴ and R⁵ are each 2,4,6-trimethylphenyl.

Aspect 16: A method of preparing the Ru complex catalyst **II** as listed in aspect 4,
(1) Under the protection of inert gas, p-toluenesulfonyl hydrazone and sodium ethoxide (NaOEt) or sodium methoxide (NaOMe) in ethanol are added together to produce ortho-alkoxycarbene, followed by reacting with RuCl₂P(Ph₃)₃ to produce the following Ru complex **V:** 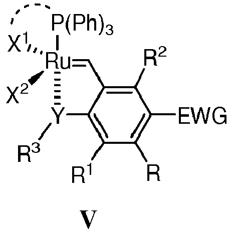 wherein X¹, X², Y, R, R¹, R², R³ and EWG each is as defined as aspects 4-9 respectively.
(2) Under the protection of inert gas, complex **V,** the product of step 1, reacts with tricyclohexyl phosphine (PCy₃) to produce the following Ru complex **VI:** 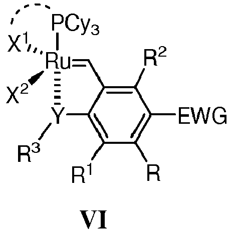 wherein X¹, X², Y, R, R¹, R², R³ and EWG each is as defined as aspects 4-9 respectively.
(3) According to the chemical activity, under the protection of inert gas, the complex **V** of step 1 or the complex **VI** of step 2 is used to produce Ru complex catalyst **II** of aspect 4.

Aspect 17: A method of preparing the immobilized Ru complex catalyst as in aspect 10, comprising the following steps:
(1) Vinylation product of ester-substituted 4-hydroxybenzensulfonamide is obtained by vinylation at ortho-position of hydroxy of 4-hydroxybenzensulfonamide;
(2) Etherification of the above vinylated ortho-phenol product (step 1) with alkyl halide offers the etherified product;;
(3) Hydrolysis of the etherified product from step 2 in basic solution offers hydrolytic product;
(4) In the presence of coupling reagent, hydrolytic product of step 3 reacts with either hydroxyl or amino group on the surface of macromolecular substance to gain immobilized ruthenium ligand;
(5) Immobilized ligand of step 4 reacts with RuCl₂ (=CHPh)(PPh₃)₂ to gain Ru intermediate product;
(6) The final product is produced by the reaction of immobilized Ru complex intermediate of step 5 with tricyclohexyl phosphine (PCy₃) ligand or another ligand H₂IMes **(IIIa).**

Aspect 18: The preparation method according to aspect 17, wherein the vinylation of step 1: under the protection of inert gas, tertiary amine (twice to thrice by volume, i.e. 2-3 BV) is added to tin tetrachloride (SnCl₄, 1 BV) and 1,2-dichloroethane (DCE, 3-6 BV) solution at -30 °C to -50°C with inlet of acetylene for 4∼6 hours, and ester-substituted 4-hydroxybenzensulfonamide is added for vinylation at room temperature, then vinylated ortho-phenol product is produced at 60∼100°C;

Step 2: the vinylated ortho-phenol product of step1 reacts with alkyl halide to offer etherification product;

Step 5: the immobilized Ru ligand of step 4 reacts with RuCl₂ (=CHPh)(H₂IMes) and CuCl in solvent, e.g., DCM, DCE, toluene, etc.

Aspect 19: The preparing method according to aspect 18, wherein the inert gas of step 1 is argon, tertiary amine (2 BV) is added to tin tetrachloride (1 BV) and 1,2-dichloroethane (3.5 BV) solution at -40°C with inlet of acetylene for 6 hours, then ester-substituted 4-hydroxybenzensulfonamide is added at room temperature to produce vinylated ortho-phenol product at 80°C;

Step 2: the vinylated ortho-phenol product of step1 reacts with isopropyl iodide in dimethyl formamide solution by etherification;

Step 3: hydrolysis is carried out in NaOH-alcohol or NaOH -H₂O solution;

Step 4: in the presence of dicyclohexylcarbodiimide (DCC), the hydrolytic product of step 3 reacts with either hydroxyl or amino group on the surface of polystyrene resin to gain the immobilized Ru ligand;

Step 5: the immobilized Ru complex ligand of step 4 reacts with RuCl₂ (=CHPh)(PPh₃) and CuCl in dichloromethane (DCM) to gain immobilized ruthenium complex.

Step 6: The final product is gained by the reaction of immobilized Ru complex of the step 5 with tricyclohexyl phosphine (PCy₃) ligand or another ligand H₂IMes **(IIIa)** in dichloromethane.

Aspect 20: Use of Ru complexes according to aspect 4 in olefin metathesis reactions as catalysts.

Aspect 21: The use according to aspect 20, wherein to carry out olefin metathesis reactions including intramolecular ring-closing olefin metathesis reactions, intermolecular olefin metathesis reactions, or olefin metathesis polymerization reactions.

Aspect 22: Use of the immobilized Ru complex catalysts according to aspect 10 in catalyzing olefin metathesis reactions.

Aspect 23: The use according to aspect 22, wherein to carry out olefin metathesis reactions include intramolecular ring-closing olefin metathesis reactions, intermolecular olefin metathesis reactions and olefin metathesis polymerization reactions.

### EXAMPLES

Based on relevant reported references (Hoveyda et al, US 20020107138 A1, US 6921735 B2, J. Am. Chem. Soc. 1999, 121, 791-799, and J. Am. Chem. Soc. 2000, 122, 8168-8179) in the present invention, a serious of ruthenium complexes **7a-n, 9a-j, 34a** and **35a-b** which are substituted by various electron-withdrawing groups are prepared respectively, and new immobilized Ru complex catalysts **18a-b** and **19a-b** which are in possession of catalytic activity and easy to recycle are also synthesized. Moreover, the present invention researches two new preparation methods aimed at different 5-EWG substituted groups.

The following is synthesis of Ruthenium Complexes with 3-EWG-2-alkoxybenzylidene ligands **5a-5i:**

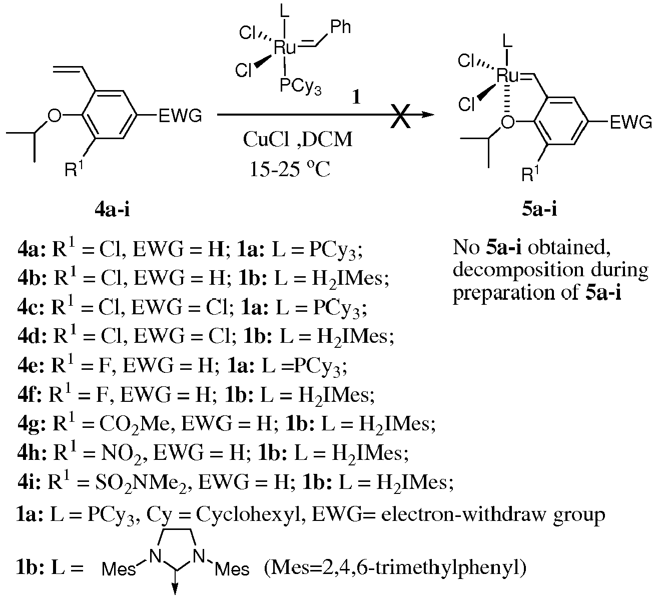

### Example 1

### Synthesis of 1-Chloro-2-isopropoxy-3-vinyl-benzene and Corresponding Ruthenium Complexes 5a, 5b

SnCl₄ (36ml 25mL, 0.2 mol) and 1,2- dichloroethane(240ml 200ml) were added into a 1 L three-necked flask with drop funnel, stirrer bar and temperature indicator under Ar atmosphere and cooled to -50°C by dry ice acetone bath. Keep temperature at -50°C, tributylamine (72ml 50ml, 0.2mol) was added and stirred for 1 hr. Followed by inletting acetylene at -50°C for 6 hr, 2-chorophenol (6.50g, 50mmol) was added at room temperature. After finishing the addition, the mixture was heated at 70°C for 2 hrs, and ortho-vinylphenol was obtained.

After the reaction was completed, K₂CO₃ (25g) and methanol (100ml) were added and heated to 60°C for 1 hr, dropped HCl (2N) with ice baths to adjust pH value lower than 2. Extracted with ethyl acetate (2 × 300ml), dried over Na₂SO₄, then the majority of ethyl acetate was removed by rotovap, extracted with the mixed solution of ethyl acetate and petroleum ether(1:1, 2×300ml), dried over Na₂SO₄, the mixed solvent was removed by rotovap, 4.83g of ortho-vinylphenol was obtained by silica gel column chromatography(yield: 63%, purity: 98%).

It is certified that the product is ligand of Ru complex. ¹H NMR (400 MHz, CDCl₃: δ= 7.26 ppm): 7.87 (d,1H, J=2.35Hz), 7.59 (dd, 1H, J=2.35, 8.22Hz), 7.14 (m, 2H), 6.86 (m, 2H), 5.82 (d, 1H, J=17.22Hz), 5.44 (d, 1H, J=11.35 Hz) (s = singlet, d = doublet, t = triplet, q = quartet, br = broad, m = multiplet). Molecular weight (M+H⁺): *m*/*z* calculated: 250.05, found: 250.1.

Vinylated product (1.55g, 10 mmol) and 2-iodopropance (1.5ml, 15 mmol, 1.5 equiv) were dissolved in DMF (15ml), followed by adding K₂CO₃ (3.9g, 30 mmol) to DMF solution, then heated at 65°C overnight (15 hrs), monitored by HPLC until completed. After removing DMF, the mixture was washed with water, The aqueous layer was extracted with Et₂O (2×100ml), and the combined organic layers were dried, and purified. The yellow solid product was obtained by purification. The product was purified by silica gel column chromatography to offer 1.69g of etherified product **4a** (yield,: 82%,purity: 98%).

It is certified that the ligand of Ru complex, ¹HNMR (CDCl₃: δ = 7.26 ppm): 7.42 (dd, 1H, J = 1.56, 7.82Hz), 7.29 (dd, 1H, J = 1.56, 7.83Hz), 7.02 (m, 2H), 5.73 (d, 1H, J = 17.60Hz), 5.56 (d, 1H, J = 11.34Hz), 4.43 (m, 1H), 1.32 (d, 6H, J = 6.26Hz). (M+H⁺) : *m*/*z* calculated: 197.1, found: 197.2.

Based on reference for the preparation of complexes (Hoveyda et al, J. Am. Chem. Soc. 2001, 123, 749), Ru complex **1a** (290 mg, 0.30 mmol) and CuCl (75 mg, 0.75mmol) were added into a round-bottom flask under Ar atmosphere and dissolved in dichloromethane (DCM 5.0 ml). Ru complex ligand 4a (60mg, 0.30mmol) dissolved in DCM (1.0ml) was added into reaction system. The mixture was stirred for 30 min at room temperature, and then the reaction was completed. It is surprising that there were no any raw material in the solution; however, no any molecular ion peak was observed by Mass spectrograph (MS), which means corresponding complex product **5a** can not be obtained during complexation reaction. And no any purple product of complex **5a** was found and observed by TLC.

### Example 2

### Synthesis of Ruthenium complex 5b

More stable Ru complex **1b** (260mg, 0.30mmol) instead of **1a** and CuCl (75mg, 0.75mmol) were added into round-bottom flask under an Ar and dissolved in DCM (5.0 ml), and Ru complex ligand 4a (60mg, 0.30mmol) dissolved in DCM (1.0ml) was added into the reaction system. After stirred for 30 min at 20°C, the reaction was completed and no any **1b** left in the reaction solution. However, no and molecular ion peak was observed by Mass spectrograph (MS), which means corresponding complex product **5b** can not be obtained during complexation reaction. And no any green product of **5a** was found and observed by TLC.

### Example 3

### Synthesis of Ruthenium complex 5e-5i

Other substituents such as Fluoro **(4e),** ester **(4g),** nitryl **(4h),** aminosulfonyl **(4i)** etc. instead of chloro group as ortho-position of isopropoxy group still do not obtain any corresponding complexes **5e-5i** which was proved by the fact that no any molecular ion peak was found and observed by Mass spectrograph (MS), and no any green color for the product of **5e-5i** was observed by TLC. The result is that if the group located in the ortho- position of isopropoxy group is electron-withdrawing group such as halogen atom, ester, nitro, aminosulfonyl group, the substituted styrene ligands can not form stable Ru complexes during complexation reaction. It shows that although Hoveyda et al. first prepared Ru complexes **10a** and **10b** produced from isopropoxy styrene, but no further study was made to study the significant effect of different substituents on stability of corresponding Ru complexes.

It was confirmed by the experimental results, the preferable substitutional position of electron-withdrawing groups such as chloro, fluoro, ester, nitryl and aminosulfonyl group in the para-position of isopropoxy group instead of the ortho-position. This result is not consistent in general electronic effect, and so far it is difficult to explain why chloro group is much more stable at the pare-position of isopropoxy group than at ortho-position.

The following is synthesis of Ruthenium complexes with 5-EWG-2-alkoxybenzylidene ligands **7a-k, 7m,** and **7n:**

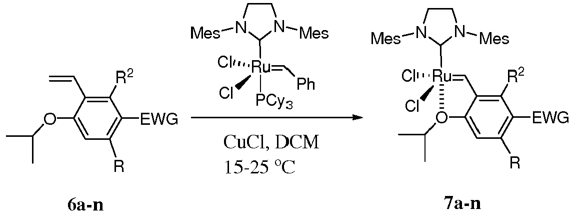

The structures of complexes **7a-k, 7m, 7n:**

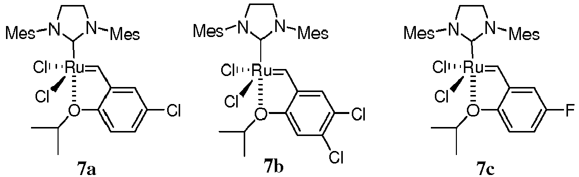

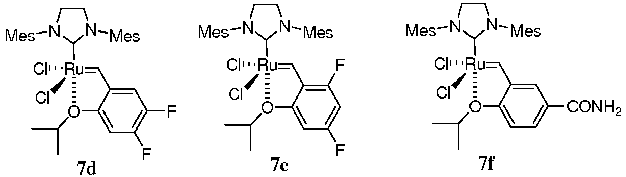

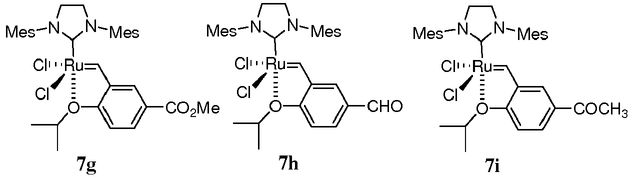

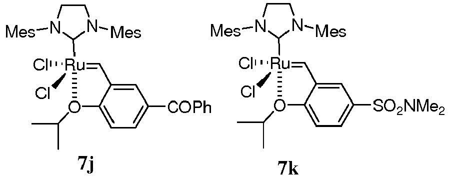

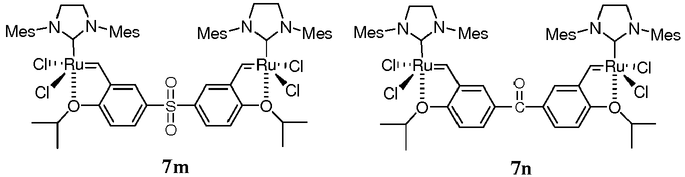

### Example 4

### Synthesis of Ru Complex 7a

Ru complex (H₂IMES)(PCy₃)Cl₂Ru=CHPh (formula **1b,** 450mg, 0.5mmol) and CuCl (135 mg, 1.25mmol, 2.5 eq) were added into a round-bottom flask under an Ar and dissolved in DCM (5.0 ml), and complex ligand **(6a,** 105mg, 0.5mmol, 1.0 eq) dissolved in DCM (1.0 ml) was added into the solution, and then stirred for 30 min at room temperature. After the reaction was completed, hexane (5ml) was added, then filtered. The filtrate was concentrated and deposited from added methanol (10ml), then filtered, washed with methanol (3×3ml) and purified by silica gel column chromatography to give 223mg of green solid product (yield: 68%, purity: 98%).

The Ru complex **13** was confirmed by ¹HNMR and MS analysis: ¹HNMR (400 MHz, CDCl₃): δ = 16.44 (s, 1H, Ru=CH), 7.46 (dd, 1H, J = 2.74, 9.00Hz), 7.08 (s, 4H), 6.89 (d, 1H, J = 2.74Hz), 6.72 (d, 1H, J = 8.61Hz), 4.85 (m, 1H), 2.46 (s, 12H), 2.41 (s, 6H), 1.25 (d, 6H, J = 6.26 Hz). (M+H⁺): *m*/*z* calculated: 661.1; found: 661.2.

### Example 5

### Synthesis of Ru Complex 7b

Ru complex (H₂IMES)(PCy₃)Cl₂Ru=CHPh **(1b,** 450mg, 0.5mmol) and CuCl (135 mg, 1.25mmol, 2.5 eq) were added into a round-bottom flask under an Ar and dissolved in DCM (5.0 ml), and complex ligand **(6b,** 0.5mmol, 1.0 eq) dissolved in DCM (1.0 ml) was added into the solution. The synthetic procedure is the same as in Example **4.** 203mg of green solid product **7b** was obtained after complexation and purification (yield: 56%, purity: 97%).

The Ru complex **7b** was confirmed by ¹HNMR and MS analysis: ¹HNMR (400 MHz, CDCl₃): δ = 16.37 (s, 1H, Ru=CH), 7.07 (s, 4H), 6.98 (s, 1H), 6.88 (s, 1H), 4.82 (m, 1H), 4.18 (s, 4H), 2.45 (s, 12H), 2.40 (s, 6H), 1.25 (d, 6H, J = 6.26Hz). (M+H⁺): *m*/*z* calculated: 695.1; founded: 695.2.

### Example 6

### Synthesis of Ru Complex 7c

Ru complex (H₂IMES)(PCy₃)Cl₂Ru=CHPh **(1b,** 450mg, 0.5mmol) and CuCl (135 mg, 1.25mmol, 2.5 eq) were added into a round-bottom flask under an Ar and dissolved in DCM (5.0 ml), and complex ligand (**6c,** 0.5mmol, 1.0 eq) dissolved in DCM (1.0 ml) was added into the solution. The synthetic procedure is the same as in Example **4.** 198mg of green solid product **7c** was obtained after complexation and purification (yield: 63%, purity: 97%).

¹HNMR (300 MHz, CDCl₃): δ = 16.49 (s, 1H, Ru=CH), 7.26-7.20 (m, 1H), 7.13 (s, 4H), 6.71 (dd, J = 3.0, 9.0 Hz, 1H), 6.62 (dd, J = 3.1, 7.9 Hz, 1H), 4.85-4.81 (m, 1H, OCHMe₂), 4.19 (s, 4H), 2.47 (s,12H), 2.27 (s, 6H), 1.26 (d, J = 6.2 Hz, 6H). ¹⁹F-NMR (300 MHz, CDCl₃): δ = -41.66.

### Example 7

### Synthesis of Ru Complex 7d

Ru complex (H₂IMES)(PCy₃)Cl₂Ru=CHPh **(1b,** 450mg, 0.5mmol) and CuCl (135 mg, 1.25mmol, 2.5 eq) were added into a round-bottom flask under an Ar and dissolved in DCM (5.0 ml), and complex ligand (**6d,** 0.5mmol, 1.0 eq) dissolved in DCM (1.0 ml) was added into the solution. The synthetic procedure is the same as in Example **4.** 173mg of green solid product **7d** was obtained after complexation and purification (yield: 51%, purity: 97%).

¹HNMR (300 MHz, CDCl₃): δ = 16.21 (s, 1H, Ru=CH), 7.07 (s, 4H), 6.72 (t, J = 9.4 Hz, 1H), 6.65-6.59 (m, 1H), 4.78-4.74 (m, 1H, OCHMe₂), 4.17 (s, 4H), 2.45 (s, 12H), 2.40 (s, 6H), 1.23 (d, J = 6.1 Hz, 6H).

### Example 8

### Synthesis of Ru Complex 7e

Ru complex (H₂IMES)(PCy₃)Cl₂Ru=CHPh **(1b,** 450mg, 0.5mmol) and CuCl (135 mg, 1.25mmol, 2.5 eq) were added into a round-bottom flask under an Ar and dissolved in DCM (5.0 ml), and complex ligand (**6e,** 0.5mmol, 1.0 eq) dissolved in DCM (1.0 ml) was added into the solution. The synthetic procedure is the same as in Example **4**.152mg of green solid product **7e** was obtained after complexation and purification (yield: 44%, purity: 98%).

¹H NMR (300 MHz, CDCl₃): δ = 16.72 (s, 1H), 7.27 (s, 1H), 7.06 (s, 4H), 6.32 (t, 1H, J = 10.15 Hz) / 6.36-6.28 (m, 2H), 4.80 (m, 1H), 4.18 (s, 4H), 2.47 (s, 12H), 2.37 (s, 6H), 1.28 (d, 6H, J = 6.23 Hz).

### Example 9

### Synthesis of Ru Complex 7f

Ru complex (H₂IMES)(PCy₃)Cl₂Ru=CHPh **(1b,** 450mg, 0.5mmol) and CuCl (135 mg, 1.25mmol, 2.5 eq) were added into a round-bottom flask under an Ar and dissolved in DCM (5.0 ml), and complex ligand (**6f,** 0.5mmol, 1.0 eq) dissolved in DCM (1.0 ml) was added into the solution. The synthetic procedure is the same as in Example **4.** 213mg of green solid product **7e** was obtained after complexation and purification (yield: 63%, purity: 98%).

¹H-NMR (300 MHz, CDCl₃): δ = 16.55 (s, 1H, Ru=CH), 7.93 (d, *J* = 6.9 Hz, 1H), 7.34 (d, J = 1.4 Hz, 1H), 7.09 (s, 4H), 6.81 (d, J = 8.8 Hz, 1H), 4.94-4.90 (m, 1H, OCHMe₂), 4.19 (s, 4H), 2.47 (s,12H), 2.42 (s, 6H), 1.27 (d, *J=* 5.9 Hz, 6H).

### Example 10

### Synthesis of Ru Complex 7g

Ru complex (H₂IMES)(PCy₃)Cl₂Ru=CHPh **(1b,** 450mg, 0.5mmol) and CuCl (135 mg, 1.25mmol, 2.5 eq) were added into a round-bottom flask under an Ar and dissolved in DCM (5.0 ml), and complex ligand (**6g,** 0.5mmol, 1.0 eq) dissolved in DCM (1.0 ml) was added into the solution. The synthetic procedure is the same as in Example **4.** 197mg of green solid product **7g** was obtained after complexation and purification (yield: 56%, purity: 98%).

¹HNMR (300 MHz, CDCl₃): δ = 16.45 (s, 1H, Ru=CH), 8.20 (dd, J = 2.2, 8.8 Hz, 1H), 7.63 (d, J = 2.2 Hz, 1H), 7.09 (s, 4H), 6.84 (d, J = 8.8 Hz, 1H), 4.97-4.93 (m, 1H, OCHMe₂), 4.20 (s, 4H), 3.90 (s, 3H), 2.47 (s,12H), 2.43 (s, 6H), 1.29 (d, J = 6.2 Hz, 6H).

### Example 11

### Synthesis of Ru Complex 7h

Ru complex (H₂IMES)(PCy₃)Cl₂Ru=CHPh **(1b,** 450mg, 0.5mmol) and CuCl (135 mg, 1.25mmol, 2.5 eq) were added into a round-bottom flask under an Ar and dissolved in DCM (5.0 ml), and complex ligand (**6h,** 0.5mmol, 1.0 eq) dissolved in DCM (1.0 ml) was added into the solution. The synthetic procedure is the same as in Example **4.** 178mg of green solid product **7h** was obtained after complexation and purification (yield: 52%, purity: 98%).

¹HNMR (300 MHz, CDCl₃): δ = 16.61 (s, 1H, Ru=CH), 9.89 (s, 1H, CHO), 8.17 (dd, J = 2.2, 8.8 Hz, 1H), 7.44 (d, J = 2.2 Hz, 1H), 7.09 (s, 4H), 6.95 (d, J = 8.8 Hz, 1H), 5.01-4.97 (m, 1H, OCHMe₂), 4.19 (s, 4H), 2.47 (s,12H), 2.43 (s, 6H), 1.31 (d, J = 6.3 Hz, 6H).

### Example 12

### Synthesis of Ru Complex 7i

Ru complex (H₂IMES)(PCy₃)Cl₂Ru=CHPh **(1b,** 450mg, 0.5mmol) and CuCl (135 mg, 1.25mmol, 2.5 eq) were added into a round-bottom flask under an Ar and dissolved in DCM (5.0 ml), and complex ligand (**6i,** 0.5mmol, 1.0 eq) dissolved in DCM (1.0 ml) was added into the solution. The synthetic procedure is the same as in Example **4.** 189mg of green solid product **7i** was obtained after complexation and purification (yield: 55%, purity: 98%).

¹HNMR (300 MHz, CDCl₃): δ = 16.49 (s, 1H, Ru=CH), 8.16 (dd, J = 1.9, 8.8 Hz, 1H), 7.53 (d, J = 1.9 Hz, 1H), 7.09 (s, 4H), 6.87 (d, J = 8.8 Hz, 1H), 4.98-4.94 (m, 1H, OCHMe₂), 4.21 (s, 4H), 2.52 (s, 3H), 2.48 (s,12H), 2.43 (s, 6H), 1.29 (d, J = 5.9 Hz, 6H).

### Example 13

### Synthesis of Ru Complex 7j

Ru complex (H₂IMES)(PCy₃)Cl₂Ru=CHPh **(1b,** 450mg, 0.5mmol) and CuCl (135 mg, 1.25mmol, 2.5 eq) were added into a round-bottom flask under an Ar and dissolved in DCM (5.0 ml), and complex ligand (**6j,** 0.5mmol, 1.0 eq) dissolved in DCM (1.0 ml) was added into the solution. The synthetic procedure is the same as in Example **4.** 199mg of green solid product **7j** was obtained after complexation and purification (yield: 53%, purity: 97%).

¹HNMR (300 MHz, CDCl₃): δ = 16.39 (s, 1H, Ru=CH), 8.10 (dd, J = 1.8, 8.4 Hz, 1H), 7.75-7.72 (m, 2H), 7.63-7.58 (m, 1H), 7.52-7.47 (m, 2H), 7.35 (d, J = 1.8 Hz, 1H), 7.02 (s, 4H), 6.92 (d, J = 8.4 Hz, 1H), 5.01-4.97 (m, 1H, OCHMe₂), 4.19 (s, 4H), 2.46 (s, 12H), 2.24 (s, 12H), 1.29 (d, J = 8.1 Hz, 6H).

### Example 14

### Synthesis of Ru Complex 7k

Ru complex (H₂IMES)(PCy₃)Cl₂Ru=CHPh **(1b,** 450mg, 0.5mmol) and CuCl (135 mg, 1.25mmol, 2.5 eq) were added into a round-bottom flask under an Ar and dissolved in DCM (5.0 ml), and complex ligand (**6k,** 0.5mmol, 1.0 eq) dissolved in DCM (1.0 ml) was added into the solution. The synthetic procedure is the same as in Example **4.** 247mg of green solid product **7k** was obtained after complexation and purification (yield: 66%, purity: 98%).

¹HNMR (400 MHz, CDCl₃): δ = 16.39 (s, 1H, Ru=CH), 7.93 (dd, J = 2.2, 8.8 Hz, 1H), 7.32 (d, J = 2.2 Hz, 1H), 7.08 (s, 4H), 6.91 (d, **J** = 8.8 Hz, 1H), 4.97-4.94 (m, 1H, OCHMe₂), 4.21 (s, 4H), 2.71 (s, 6H), 2.46 (s,12H), 2.40 (s, 6H), 1.29 (d, J = 5.9 Hz, 6H).

### Example 15

### Synthesis of Ru Complex 7m

Ru complex (H₂IMES)(PCy₃)Cl₂Ru=CHPh **(1b,** 450mg, 0.5mmol) and CuCl (135 mg, 1.25mmol, 2.5 eq) were added into a round-bottom flask under an Ar and dissolved in DCM (5.0 ml), and complex ligand (**6m,** 0.5mmol, 1.0 eq) dissolved in DCM (1.0 ml) was added into the solution. The synthetic procedure is the same as in Example **4.** 247mg of green solid product **7m** was obtained after complexation and purification (yield: 56%, purity: 97%).

¹HNMR (300 MHz, CDCl₃): δ = 16.42 (s, 2H, Ru=CH), 7.87 (dd, J = 2.2, 8.8 Hz, 2H), 7.53 (d, J = 2.2 Hz, 2H), 7.07 (s, 8H), 6.87 (d, J = 8.8 Hz, 2H), 4.96-4.92 (m, 2H, OCHMe₂), 3.15 (s, 8H), 2.45 (s, 24H), 2.41 (s, 12H), 1.27 (d, J = 5.9 Hz, 12H).

### Example 16

### Synthesis of Ru Complex 7n

Ru complex (H₂IMES)(PCy₃)Cl₂Ru=CHPh **(1b,** 450mg, 0.5mmol) and CuCl (135 mg, 1.25mmol, 2.5 eq) were added into a round-bottom flask under an Ar and dissolved in DCM (5.0 ml), and complex ligand (**6n,** 0.5mmol, 1.0 eq) dissolved in DCM (1.0 ml) was added into the solution. The synthetic procedure is the same as in Example **4.** 171mg of green solid product **7n** was obtained after complexation and purification (yield: 52%, purity: 97%).

¹HNMR (300 MHz, CDCl₃): δ = 16.44 (s, 2H, Ru=CH), 7.93 (dd, J = 2.0, 8.4 Hz, 2H), 7.30 (d, J = 2.0 Hz, 2H), 7.03 (s, 8H), 6.88 (d, **J** = 8.4 Hz, 2H), 5.01-4.97 (m, 2H, OCHMe₂), 4.19 (s, 8H), 2.47 (s, 24H), 2.26 (s, 12H), 1.33 (d, J = 6.2 Hz, 12H).

The following is synthesis of Ruthenium Complexes with 5-(R₂NSO₂)-2-alkoxybenzylidene ligands **9a-9j:**

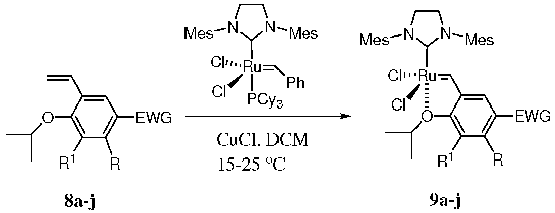

The structural formula **9a-j** are as follows:

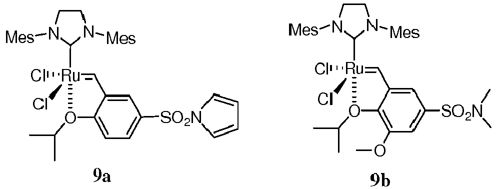

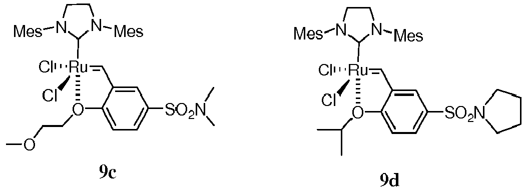

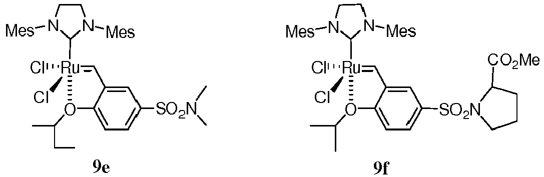

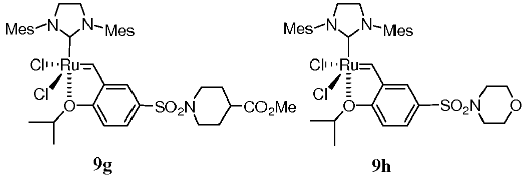

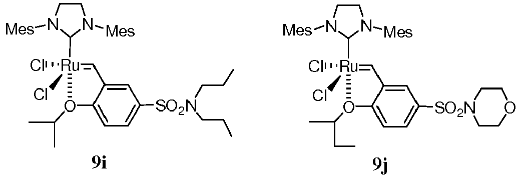

### Example 17

### Synthesis of Ru Complex 9a

Ru complex (H₂IMES)(PCy₃)Cl₂Ru=CHPh **(1b,** 450mg, 0.5mmol) and CuCl (135 mg, 1.25mmol, 2.5 eq) were added into a round-bottom flask under an Ar and dissolved in DCM (5.0 ml), and complex ligand (**8a,** 0.5mmol, 1.0 eq) dissolved in DCM (1.0 ml) was added into the solution. The synthetic procedure is the same as in Example **4.** 211mg of green solid product **9a** was obtained after complexation and purification (yield: 62%, purity: 97%).

¹HNMR (400 MHz, CDCl₃): δ = 16.36 (s, 1H, Ru=CH), 7.98 (dd, 1H, J = 2.35, 8.81Hz), 7.40 (d, 1H, J = 2.35Hz), 7.10 (m, 2H), 7.08 (s, 4H), 6.87 (d, 1H, J = 9.00Hz), 6.31 (m,2H), 4.92 (m, 1H, OCHMe₂), 4.20 (s, 4H), 2.44 (s, 18H), 1.13 (d, 6H, J = 5.87Hz).

### Example 18

### Synthesis of Ru Complex 9b

Ru complex (H₂IMES)(PCy₃)Cl₂Ru=CHPh **(1b,** 450mg, 0.5mmol) and CuCl (135 mg, 1.25mmol, 2.5 eq) were added into a round-bottom flask under an Ar and dissolved in DCM (5.0 ml), and complex ligand (**8b,** 0.5mmol, 1.0 eq) dissolved in DCM (1.0 ml) was added into the solution. The synthetic procedure is the same as in Example **4.** 158mg of green solid product **9b** was obtained after complexation and purification (yield: 41%, purity: 97%).

¹HNMR (300 MHz, CDCl₃): δ = 16.34 (s, 1H, Ru=CH), 7.45 (d, 1H, J = 1.83 Hz), 7.17 (s, 4H), 6.92 (d, 1H, J = 2.20 Hz,), 5.80 (m, 1H, OCHMe₂), 4.20 (s, 4H), 3.81 (s, 3H), 2.73 (s, 6H), 2.47 (s, 12H), 2.40 (s, 6H), 1.31 (d, 6H, J = 6.22 Hz).

### Example 19

### Synthesis of Ru Complex 9c

Ru complex (H₂IMES)(PCy₃)Cl₂Ru=CHPh **(1b,** 450mg, 0.5mmol) and CuCl (135 mg, 1.25mmol, 2.5 eq) were added into a round-bottom flask under an Ar and dissolved in DCM (5.0 ml), and complex ligand (**8c,** 0.5mmol, 1.0 eq) dissolved in DCM (1.0 ml) was added into the solution. The synthetic procedure is the same as in Example **4.** 165mg of green solid product **9c** was obtained after complexation and purification (yield: 44%, purity: 97%).

¹HNMR (300 MHz, CDCl₃): δ = 16.37(s, 1H, Ru=CH), 7.94 (dd, 1H, J = 2.20, 8.79 Hz), 7.29 (d, 1H, J = 2.20 Hz), 7.09 (s, 4H), 7.06 (d, 1H, J = 8.79 Hz), 4.34 (t, 2H, J = 5.85 Hz), 4.18 (s, 4H), 3.61 (t, 2H, J = 5.94 Hz), 3.13 (s, 3H), 2.70 (s, 6H), 2.47 (s, 12H), 2.42 (s, 6H).

### Example 20

### Synthesis of Ru Complex 9d

Ru complex (H₂IMES)(PCy₃)Cl₂Ru=CHPh **(1b,** 450mg, 0.5mmol) and CuCl (135 mg, 1.25mmol, 2.5 eq) were added into a round-bottom flask under an Ar and dissolved in DCM (5.0 ml), and complex ligand (**8d,** 0.5mmol, 1.0 eq) dissolved in DCM (1.0 ml) was added into the solution. The synthetic procedure is the same as in Example **4.** 195mg of green solid product **9d** was obtained after complexation and purification (yield: 54% purity: 97%).

¹HNMR (400 MHz, CDCl₃): δ = 16.39 (s, 1H, Ru=CH), 7.97 (dd, 1H, J = 2.35, 8.61Hz), 7.37 (d, 1H, J = 1.96Hz), 7.08 (s, 4H), 6.90 (d, 1H, J = 9.00Hz), 4.95 (m, 1H, OCHMe₂), 4.21 (s, 4H), 3.21 (m, 4H), 2.46 (s, 12H), 2.41 (s, 6H), 1.83 (m, 4H), 1.29 (d, 6H, J = 5.87Hz).

### Example 21

### Synthesis of Ru Complex 9e

Ru complex (H₂IMES)(PCy₃)Cl₂Ru=CHPh **(1b,** 450mg, 0.5mmol) and CuCl (135 mg, 1.25mmol, 2.5 eq) were added into a round-bottom flask under an Ar and dissolved in DCM (5.0 ml), and complex ligand (**8e,** 0.5mmol, 1.0 eq) dissolved in DCM (1.0 ml) was added into the solution. The synthetic procedure is the same as in Example **4.** 176mg of green solid product **9e** was obtained after complexation and purification (yield: 47%, purity: 97%).

¹HNMR (300 MHz, CDCl₃): δ = 16.40 (s, 1H, Ru=CH), 7.93 (dd, 1H, J = 2.20, 8.79 Hz), 7.33 (d, 1H, J = 2.19 Hz), 7.08 (s, 4H), 6.87 (d, 1H, J = 8.79 Hz), 4.66 (m, 1H, OCHMe₂), 4.21 (s, 4H), 2.72 (s, 6H), 2.47 (s, 12H), 2.42 (s, 6H), 1.45 (m, 2H), 1.27 (d, 3H, J = 5.86 Hz), 0.80 (t, 3H, J = 7.69 Hz).

### Example 22

### Synthesis of Ru Complex 9f

Ru complex (H₂IMES)(PCy₃)Cl₂Ru=CHPh **(1b,** 450mg, 0.5mmol) and CuCl (135 mg, 1.25mmol, 2.5 eq) were added into a round-bottom flask under an Ar and dissolved in DCM (5.0 ml), and complex ligand (**8f,** 0.5mmol, 1.0 eq) dissolved in DCM (1.0 ml) was added into the solution. The synthetic procedure is the same as in Example **4.** 196mg of green solid product **9f** was obtained after complexation and purification (yield: 52%, purity: 97%).

¹HNMR(400 MHz, CDCl₃): δ = 16.39 (s, 1H, Ru=CH), 8.04 (dd, 1H, J = 1.95, 8.60 Hz), 7.41 (d, 1H, J = 2.35 Hz), 7.10 (s, 4H), 6.89 (d, 1H, J = 8.61 Hz), 4.95 (m, 1H, OCHMe₂), 4.24 (m, 1H), 4.21 (s, 4H), 3.66 (s, 3H), 3.48 (m, 1H), 3.24 (m, 1H), 2.46 (s, 12H), 2.42 (s, 6H), 1.81-2.13 (m, 5H), 1.28 (d, 6H, J = 5.87 Hz).

### Example 23

### Synthesis of Ru Complex 9g

Ru complex (H₂IMES)(PCy₃)Cl₂Ru=CHPh **(1b,** 450mg, 0.5mmol) and CuCl (135 mg, 1.25mmol, 2.5 eq) were added into a round-bottom flask under an Ar and dissolved in DCM (5.0 ml), and complex ligand (**8g,** 0.5mmol, 1.0 eq) dissolved in DCM (1.0 ml) was added into the solution. The synthetic procedure is the same as in Example **4.** 226mg of green solid product **9g** was obtained after complexation and purification (yield: 56%, purity: 98%).

¹HNMR (300 MHz, CDCl₃): δ = 16.39 (s, 1H, Ru=CH), 7.90 (dd, 1H, J = 2.20, 8.79 Hz), 7.30 (d, 1H, J = 1.83 Hz), 7.08 (s, 4H), 6.90 (d, 1H, J = 8.79 Hz,), 4.95 (m, 1H, OCHMe₂), 4.21 (s, 4H), 3.69 (s, 3H), 3.63 (m, 1H), 2.47 (s, 12H), 2.41 (s, 6H), 2.09 (dd, 4H, J = 3.29, 13.55 Hz), 1.85 (m, 4H), 1.30 (d, 6H , J = 6.22 Hz).

### Example 24

### Synthesis of Ru Complex 9h

Ru complex (H₂IMES)(PCy₃)Cl₂Ru=CHPh **(1b,** 450mg, 0.5mmol) and CuCl (135 mg, 1.25mmol, 2.5 eq) were added into a round-bottom flask under an Ar and dissolved in DCM (5.0 ml), and complex ligand (**8h,** 0.5mmol, 1.0 eq) dissolved in DCM (1.0 ml) was added into the solution. The synthetic procedure is the same as in Example **4.** 193mg of green solid product **9h** was obtained after complexation and purification (yield: 52%, purity: 98%).

¹HNMR (300 MHz, CDCl₃): δ = 16.31 (s, 1H, Ru=CH), 7.83 (dd, 1H, J = 2.19, 8.79 Hz), 7.24 (d, 1H, J = 2.20 Hz), 7.00 (s, 4H), 6.85 (d, 1H, J = 8.79 Hz), 4.89 (m, 1H, OCHMe₂), 4.13 (s, 4H), 3.68 (t, 4H, J = 4.77 Hz), 2.95 (t, 4H, J = 4.76 Hz), 2.39 (s, 12H), 2.33 (s, 6H), 1.23 (d, 6H, J = 6.23 Hz).

### Example 25

### Synthesis of Ru Complex 9i

Ru complex (H₂IMES)(PCy₃)Cl₂Ru=CHPh **(1b,** 450mg, 0.5mmol) and CuCl (135 mg, 1.25mmol, 2.5 eq) were added into a round-bottom flask under an Ar and dissolved in DCM (5.0 ml), and complex ligand (**8i,** 0.5mmol, 1.0 eq) dissolved in DCM (1.0 ml) was added into the solution. The synthetic procedure is the same as in Example **4.** 216mg of green solid product **9i** was obtained after complexation and purification (yield: 54%, purity: 97%).

¹HNMR (300 MHz, CDCl₃): δ = 16.36 (s, 1H, Ru=CH), 7.90 (dd, 1H, J = 2.20, 8.79 Hz), 7.32 (d, 1H, J = 2.20Hz), 7.09 (s, 4H), 6.88 (d, 1H, J = 8.78 Hz), 4.66 (m, 1H, OCHMe₂), 4.21 (s, 4H), 3.77 (t, 4H, J = 4.76 Hz), 3.03 (t, 4H, J = 4.84), 2.47 (s, 12H), 2.42 (s, 6H), 1.38 (m, 2H), 1.30 (d, 3H, J = 9.15 Hz), 0.90 (t, 3H, J = 7.69 Hz).

### Example 26

### Synthesis of Ru Complex 9j

Ru complex (H₂IMES)(PCy₃)Cl₂Ru=CHPh **(1b,** 450mg, 0.5mmol) and CuCl (135 mg, 1.25mmol, 2.5 eq) were added into a round-bottom flask under an Ar and dissolved in DCM (5.0 ml), and complex ligand (**8j,** 0.5mmol, 1.0 eq) dissolved in DCM (1.0 ml) was added into the solution. The synthetic procedure is the same as in Example **4.** 186mg of green solid product **9j** was obtained after complexation and purification (yield: 47%, purity: 97%).

¹HNMR (300 MHz, CDCl₃): δ = 16.36 (s, 1H, Ru=CH), 7.90 (dd, 1H, J = 2.20, 8.79 Hz), 7.32 (d, 1H, J = 2.20 Hz), 7.09 (s, 4H), 6.88 (d, 1H, J = 8.78 Hz), 4.66 (m, 1H, OCHMe₂), 4.21 (s, 4H), 3.77 (t, 4H, J = 4.76 Hz), 3.03 (t, 4H, J = 4.84), 2.47 (s, 12H), 2.42 (s, 6H), 1.48 (m, 2H), 1.30 (d, 3H, J = 9.15 Hz), 0.80 (t, 3H, J = 7.69 Hz).

In order to save and effectively reuse expensive metal ruthenium catalyst effectively, the present invention developed immobilized ruthenium complex catalysts with advantages of easy recycle and repeatedly reuse and provided a new way of cost controlling and environment protection in product industrialization.

The following is synthesis of immobilized complex catalysts **18a-b** and **19a-b:**

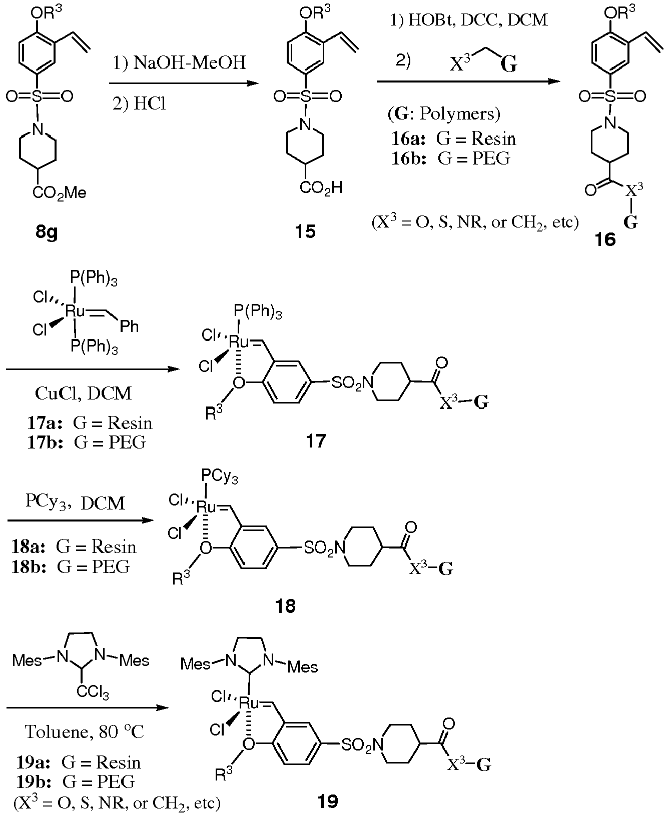

### Example 27

### Synthesis of Ru Complex ligand 15

Compound **8g** (1.8g, 4.8 mmol) was dissolved in 25mL MeOH and 10mL water, and NaOH (1.0g, 25.0mmol) was added, then the reaction mixture was stirred at room temperature for 4 hrs. The solvent was removed by rotovap. 20ml water was added and the mixture was extracted with ether (40ml) and the aqueous phase was adjusted with diluted HCl to pH = 2, then extracted with EtOAc (3×50mL) and the combined organic phase was washed with brine, dried and concentrated. 1.6g of product 15 was obtained in 92% of yield (HPLC: 98%).

¹HNMR (300 MHz, CDCl₃): δ = 7.80 (d, 1H, J = 2.47Hz), 7.60 (dd, 1H, J = 2.47, 8.79Hz), 7.00 (dd, 1H, J = 11.26, 17.85Hz), 6.95 (d, 1H, J = 8.79Hz), 5.81 (dd, 1H, J = 1.1, 17.58Hz), 5.39 (dd, 1H, J = 1.1, 11.27Hz), 4.66 (m, 1H), 3.64 (m, 2H), 2.43 (m, 2H), 2.26 (m, 1H), 2.00 (m, 2H), 1.87 (m, 2H), 1.42 (d, 6H, J = 6.05Hz). (M+H⁺): *m*/*z* calculated: 352.1, found: 352.1.

### Example 28

### Synthesis of Immobilized Ru Catalyst 19a

To the solution of compound **15** (0.80g, 2.3mmol) in DCM and HOBt was added (0.32g, 2.4mmol) under an Ar. The mixture was stirred overnight. After the reaction was completed, the mixture was filtrated and concentrated. 1.20g of product was obtained, and added to a DMF solution of polystyrene resin (0.85g, 1.44mmol, 1.0eq.) and DMAP (0.2g, 1.44mmol, 1.0eq.). The reaction mixture was stirred overnight. After the coupling was completed, the resin was washed with DMF (20ml×3), THF (20ml×3), DCM (20ml×3), 1/1 DCM/Et₂O (20ml×1), Et₂O (20ml×3) and dried to offer 0.98g of resin **16a.**

To a solution of Resin **16a** (0.90g, 1.5mmol, 1.0eq.) in DCM (15mL), (PPh₃)₂Cl₂Ru =CHPh (1.95g, 2.25mmol, 1.5eq.) and CuCl (0.39g, 3.75mmol, 2.5eq.) were added under an Ar. The solution was stirred for 2 hrs to offer product **17a,** followed by adding PCy₃ in DCM (15 mL) and stirring for 5 hrs. The resin was washed with DMF (20ml×3), THF (20ml×3), DCM (20ml×3) and dried to offer product **18a**.

To a solution of resin **18a** (0.90g, 1.5mmol, 1.0eq.) in toluene solution, H₂IMes(H)(CCl₃) (IIIa) was added. The solution was kept stirring overnight at 80°C until the reaction was completed. The resin was washed with DMF (20ml×3), THF (20ml×3), DCM (20ml×3), 1/1 DCM/Et₂O (20ml×1), Et₂O (20ml×3) (20ml) and dried to offer 1.11g of resin **19a** with Ru catalyst attached on the surface.

IR: 3454.20 (w), 2921.47 (br), 1733.20 (m), 1613.66 (s), 1112,85 (m).

### Example 29

### Synthesis of Immobilized Ru Catalyst 19b

The synthesis procedure is the same as in Example **28.** 0.36g black-green solid of Ru complex **19b** was obtained after several steps of complexation and purification.

¹HNMR (300 MHz, CDCl₃): δ = 16.38 (s, 1H, Ru=CH), 7.92 (dd, 1H, J = 2.20, 8.79 Hz), 7.30 (d, 1H, J = 1.83 Hz), 7.08 (s, 4H), 6.90 (d, 1H, J = 8.79 Hz,), 4.95 (m, 1H, OCHMe₂), 4.21 (s, 4H), 3.70-1.30 (broad peaks, PEG proton peaks overlapped).

IR: 3441.82 (w), 2925.79 (m), 1732.10 (s), 1633.66 (s), 1263.83 (s), 1106.00 (m).7

The two alternative synthesized procedures of several main classes of Ru catalysts are focus on optimizing preparation methods of all kinds of Ru catalyst, and reducing cost and resource consumption as follows. When the 5-substuent is Cl or F, triphenylphosphine ligands **28a-b** of unstable Ru complexes can be directly substituted by another ligand H₂IMes **(IIIa)** to form more stable and active Ru catalysts **30a-b.** But when 5-substuent is R₂NSO₂ and NO₂, triphenylphosphine ligands **33a-b** of unstable Ru complexes can't be directly substituted by another ligand H₂IMes **(IIIa),** and they must be after PPh₃ substituted by PCy₃ to form complexes **34a-b,** then more stable and active Ru catalysts **7k** and **10e** can be generated after PCy₃ substituted by ligand H₂IMes **(IIIa).**

The following is synthesis of Ru complexes with 5-Cl- and 5-F- substituted 2-alkoxybenzylidene ligands **30a-b:**

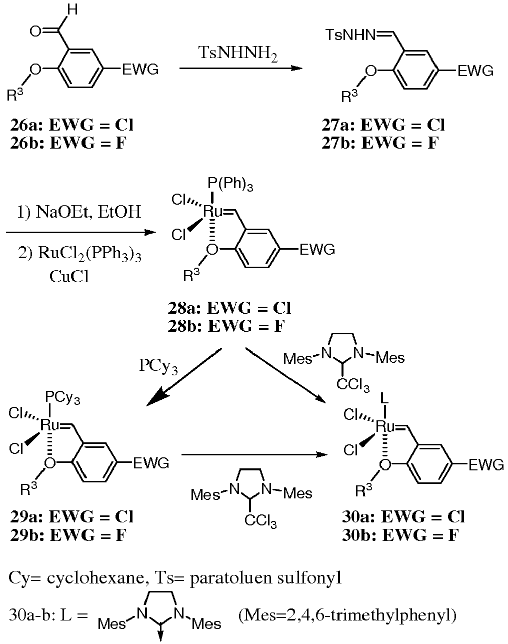

The chemical shift changes of ¹HNMR, ³¹P-NMR for products during the synthesis of complexes **30a-b** are as follows:

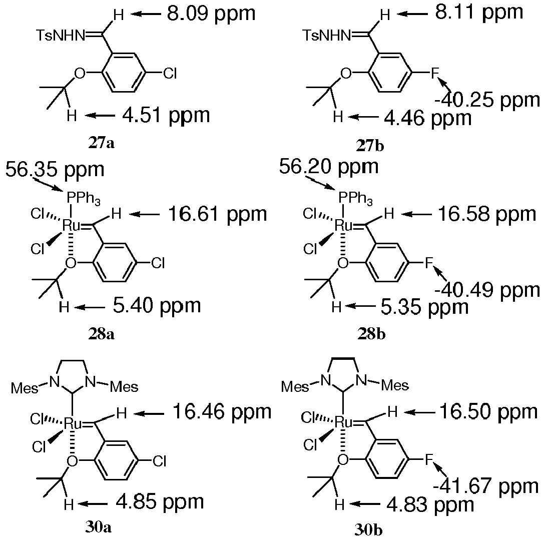

### Example 30

### Synthesis of Ru complex ligand 27a

A suspension of p-toluenesulfonyl hydrazide (26.5g, 142mmol, 1.0eq in methanol (100mL) was treated rapidly with compound **26a** (29g, 146mmol, 1.0eq with stirring. After 30 min, the solution was cooled to 0°C, and solid product was vacuum filtered, and dried to offer white crystal solid product 50.4g, (yield: 96%, HPLC purity: 99%).

¹HNMR (300 MHz, CDCl₃): δ = 8.08 (d, J = 1.6 Hz, 1H), 7.88 (d, J = 8.5 Hz, 1H), 7.77 (d, J = 2.8 Hz, 2H), 7.33 (d, J = 7.9 Hz, 1H), 7.25 (dd, J = 2.8, 7.9 Hz, 1H), 6.79 (d, J = 8.8 Hz, 2H), 4.52-4.48 (m, 1H, OCHMe₂), 2.42 (s, 3H), 1.29 (d, J = 6.1 Hz, 6H). (M+H⁺): *m*/*z* calculated: 366.1; found: 366.1.

### Example 31

### Synthesis of Ru complex 28a

Compound **27a** (10g, 27.3mmol, 1.0eq) was treated with NaOEt (5.0eq.) in EtOH (150mL) and heated to 60°C. After the reaction was completed in 30 min, water (120mL) was added, and extracted with pentane (3 × 50ml). The combined organic solution was washed with saturated Na₂CO₃ (50ml×2), brine (50ml×2), and dried with Na₂SO₄, then concentrated at 0°C to about 20mL, yield is calculated in term of 55%. Adding the concentrated diazo solution **10** (3.1g, 14.7mmol, 2.0eq.) into the RuCl₂(PPh₃)₃ (7.0g, 7.3mmol, 1.0eq solution in CH₂Cl₂ (50mL) at -78°C. After 5 min, the solution was warmed up to room temperature, and CuCl (2.9g, 29.3mmol, 4.0eq.) was added to react for another 15 min, then the reaction mixture was filtered, and the filtrate was concentrated and purified by column chromatography eluting with a gradient solvents (2:1 hexane/DCM to pure DCM). Concentration of the product fraction, which was washed with hexanes, dried under vacuum to give 2.9g of red solid powder **28a** (64% yield).

¹HNMR (300 MHz, CDCl₃): δ = 16.60 (d, J_{PH} = 6.8 Hz, 1H, Ru=CH), 7.63-7.44 (m, 17H), 7.14 (d, J = 8.5 Hz, 1H), 5.41-5.38 (m, 1H, OCHMe₂), 1.90 (d, J = 6.4 Hz, 6H). ³¹P-NMR (121 MHz, CDCl₃): δ = 56.350 (s, PPh₃).

### Example 32

### Synthesis of Ru complex 30a

H₂IMes(H)(CCl₃) (1.38g, 3.24mmol, 2.0eq.) and compound **28a** (1.0g, 1.62mmol, 1.0eq was dissolved in toluene and heated to 80°C for 1.5 hrs, then cooled to crystallize, filtered and purified. The solution was purified by column chromatography to offer dark-green solid, washed with methanol and hexane, dried under vacuum to offer 533mg of product product **30a** as green solid (51% yield).

¹HNMR (300 MHz, CDCl₃): δ = 16.46 (s, 1H, Ru=CH), 7.46 (dd, J = 2.6, 8.7 Hz, 1H), 7.08 (s, 4H), 6.89 (d, J = 2.6 Hz, 1H), 6.72 (d, J = 8.7 Hz, 1H), 4.88-4.82 (m, 1H, OCHMe₂), 4.18 (s, 4H), 2.46 (s,12H), 2.41 (s, 6H), 1.25 (d, J = 6.2 Hz, 6H).

### Example 33

### Synthesis of Ru complex 27b

The synthesis procedure is the same as in Example 30. The yield for Ru complex ligand 27b is 95% after purification and the NMR results for **27b** are as follow:

¹HNMR (300 MHz, CDCl₃): δ = 8.10 (d, J = 1.9 Hz, 1H), 7.97 (s, 1H), 7.87 (d, J = 8.2 Hz, 2H), 7.50 (dd, J = 3.0, 9.0 Hz, 1H), 7.32 (d, J = 8.2 Hz, 2H), 7.02-6.95 (m, 1H), 6.80 (dd, J = 4.4, 9.1 Hz, 1H), 4.53-4.42 (m, 1H), 2.41 (s, 3H), 1.29 (d, J = 6.1 Hz, 6H). ¹⁹F-NMR (282 MHz, CDCl₃): δ = -40.25. (M+H⁺): *m*/*z* calculated: 350.1; found: 350.2.

### Example 34

### Synthesis of Ru complex 28b

The synthesis procedure is the same as in Example 31. The yield for purple Ru complex solid **28b** is 57% after complexation and purification, and the NMR results for **28b** are as follow:

¹HNMR (300 MHz, CDCl₃): δ = 16.59 (d, J_{PH} = 6.6 Hz, 1H, Ru=CH), 7.55-7.26 (m, 17H), 7.09 (dd, J = 3.9, 9.0 Hz, 1H), 5.37-5.32 (m, 1H, OCHMe₂), 1.86 (d, J = 6.3 Hz, 6H). ¹⁹F-NMR (282 MHz, CDCl₃): δ = -40.48. ³¹P-NMR (121 MHz, CDCl₃): δ = 56.19 (s, PPh₃).

### Example 35

### Synthesis of Ru complex 30b

The synthesis procedure is the same as in Example 32. The yield for green Ru complex solid **30b** is 42% after complexation and purification, and the NMR results for **30b** are as follow:

¹HNMR (300 MHz, CDCl₃): δ = 16.49 (s, 1H, Ru=CH), 7.26-7.20 (m, 1H), 7.13 (s, 4H), 6.71 (dd, J = 3.0, 9.0 Hz, 1H), 6.62 (dd, J = 3.1, 7.9 Hz, 1H), 4.85-4.81 (m, 1H, OCHMe₂), 4.19 (s, 4H), 2.47 (s,12H), 2.27 (s, 6H), 1.26 (d, J = 6.2 Hz, 6H). ¹⁹F-NMR (282 MHz, CDCl₃): δ = -41.663.

The following is synthetic procedure of Ru complexes with 5-R₂NSO₂- and 5-NO₂-2-alkoxybenzylidene ligands **7k, 10e:**

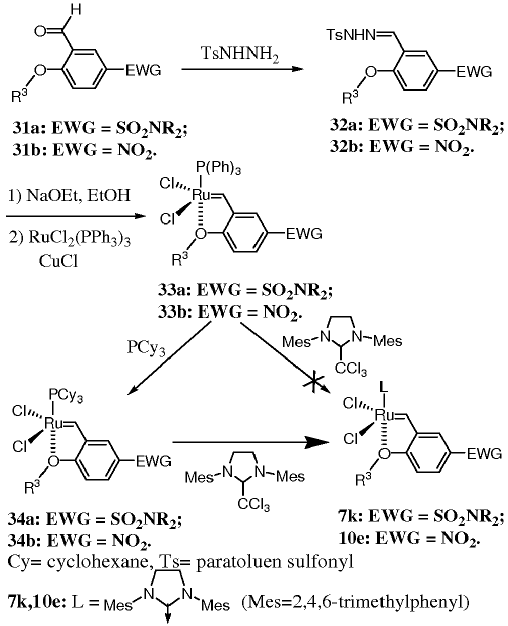

The chemical shift changes of ¹HNMR, ³¹P-NMR for product during the preparation of complexes **7k, 10e** are as follows:

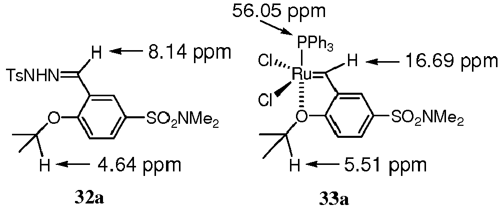

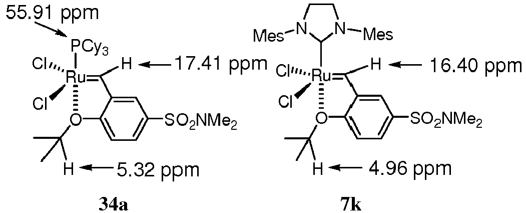

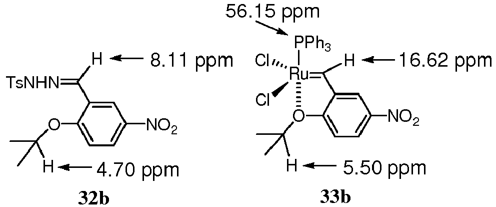

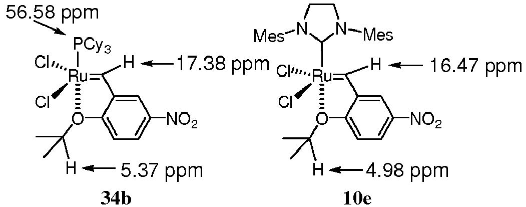

The following are the structural formula of New Ru complexes with PCy₃ ligands **34a, 35a-b:**

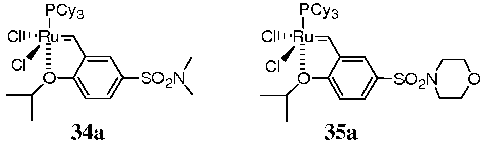

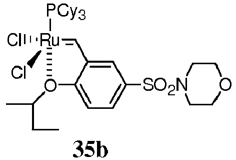

### Example 36

### Synthesis of Ru complex ligand 32a

The synthesis procedure is the same as in Example 30. After purification, the yield for Ru complex ligand **32a** is 96%, and the NMR result of **32a** is as follow:

¹HNMR (300 MHz, CDCl₃): δ = 8.14-8.11 (m, 2H), 7.87 (d, J = 8.2 Hz, 2H), 7.71-7.67 (m, 1H), 7.30 (d, J = 8.2 Hz, 2H), 6.94 (d, J = 8.8 Hz, 1H), 4.68-4.60 (m, 1H, OCHMe₂), 2.70 (s, 6H), 2.40 (s, 3H), 1.35 (d, J = 6.0 Hz, 6H). (M+H⁺): *m*/*z* calculated: 439.1; found: 439.2.

### Example 37

### Synthesis of Ru complex 33a

The synthesis procedure is the same as in Example 31. After complexation and purification, the yield for purple Ru complex solid **33a** is 63%, and the NMR results of **33a** are as follow:

¹HNMR (300 MHz, CDCl₃): δ = 16.69 (d, J_{PH} = 6.9 Hz, 1H, Ru=CH), 8.09-8.06 (m, 2H), 7.57-7.43 (m, 16H), 7.34 (d, J = 9.0 Hz, 1H), 5.53-5.49 (m, 1H, OCHMe₂), 2.82 (s, 6H), 1.94 (d, J = 6.4 Hz, 6H). ³¹P-NMR (121 MHz, CDCl₃) δ = 56.05 (s, PPh₃).

### Example 38

### Synthesis of Ru complex 34a

Complex **33a** (4.0g, 5.8mmol, 1.0eq was dissolved in CH₂Cl₂(50mL) under an Ar, then tricyclohexylphosphine (PCy₃, 3.25g, 11.6mmol, 2.0eq.) was added. The solution was stirred at 20°C for 0.5h, then concentrated and purified by column chromatography eluting with a gradient solvent (2:1 petroleum ether/DCM to DCM). Concentration was dried under vacuum to offer 2.76g of product **34a** as a purple solid (67% yield).

¹HNMR (300 MHz, CDCl₃): δ = 17.40 (d, J_{PH} = 4.3 Hz, 1H, Ru=CH), 8.13 (d, J = 2.1 Hz, 1H), 8.04 (dd, J = 2.1, 8.6 Hz, 1H), 7.21 (d, J = 8.6 Hz, 1H), 5.36-5.30 (m, 1H, OCHMe₂), 2.79 (s, 6H), 2.39-1.28 (m, 39H). ³¹P-NMR (121 MHz, CDCl₃): δ = 55.91 (s, PCy₃).

### Example 39

### Synthesis of Ru complex 7k

Complex **34a** (1.2g, 1.6mmol, 1.0eq and H₂IMes(H)(CCl₃) (1.4g, 3.2mmol, 2.0eq.) was dissolved in toluene (10mL) and heated to 80°C for 1.5h, then cooled to crystallize. After the solution was filtered and purified, 685mg of green solid **7k** was obtained (58% yield).

¹HNMR (300 MHz, CDCl₃): δ = 16.39 (s, 1H, Ru=CH), 7.93 (dd, J = 2.2, 8.8 Hz, 1H), 7.32 (d, J = 2.2 Hz, 1H), 7.08 (s, 4H), 6.91 (d, J = 8.8 Hz, 1H), 4.97-4.94 (m, 1H, OCHMe₂), 4.21 (s, 4H), 2.71 (s, 6H), 2.46 (s,12H), 2.40 (s, 6H), 1.29 (d, J = 5.9 Hz, 6H).

### Example 40

### Synthesis of Ru complex 32b

The synthesis procedure is the same as in Example 30. After purification, the yield for Ru complex ligand **32b** is 93%, and the NMR result of **32b** is as follow:

¹HNMR (300 MHz, CDCl₃): δ = 8.62 (d, J = 3.0 Hz, 1H), 8.18 (dd, J = 3.0, 9.4 Hz, 1H), 8.16 (s, 1H), 7.91 (d, J = 8.3 Hz, 2H), 7.36 (d, J = 8.3 Hz, 2H), 6.91 (d, J = 9.4 Hz, 1H), 4.74-4.66 (m, 1H, OCHMe₂), 2.42 (s, 3H), 1.38 (d, J = 6.0 Hz, 6H). (M+H⁺): m/z calculated: 378.1; found: 378.1.

### Example 41

### Synthesis of Ru complex 33b

The synthesis procedure is the same as in Example 31. After complexation and purification, the yield for purple Ru complex solid **33b** is 66%, and the NMR results of **33b** is as follow:

¹HNMR (300 MHz, CDCl₃): δ = 16.62 (d, J_{PH} = 6.8 Hz, 1H, Ru=CH), 8.53 (dd, J = 2.6, 9.0 Hz, 1H), 7.55-7.39 (m, 16H), 7.27 (d, J = 9.0 Hz, 1H), 5.52-5.47 (m, 1H, OCHMe₂), 1.91 (d, J = 6.0 Hz, 6H).

### Example 42

### Synthesis of Ru complex 34b

The synthesis procedure is the same as in Example 38. After complexation and purification, the yield for purple Ru complex solid **34b** is 71 %, and the NMR results of **34b** is as follow:

¹HNMR (300 MHz, CDCl₃): δ = 17.38 (d, J_{PH} = 4.7 Hz, 1H, Ru=CH), 8.53 (dd, J = 2.6, 8 Hz, 1H), 7.49 (m, 1H), 7.27 (d, J = 8.0 Hz, 1H), 5.37 (m, 1H, OCHMe₂), 2.35-1.26 (m, 39H).

### Example 43

### Synthesis of Ru complex 10e

The synthesis procedure is the same as in Example 39. After complexation and purification, the yield for green Ru complex solid **10e** is 61%, and the NMR results of **10e** is as follow:

¹HNMR (300 MHz, CDCl₃): δ = 16.47 (s, 1H, Ru=CH), 8.43 (dd, J = 2.5, 9.2 Hz, 1H), 7.82 (d, J = 2.5 Hz, 1H), 7.10 (s, 4H), 6.89 (d, J = 9.2 Hz, 1H), 5.01-4.95 (m, 1H, OCHMe₂), 4.22 (s, 4H), 2.46 (s,12H), 2.44 (s, 6H), 1.30 (d, J = 6.2 Hz, 6H).

### Example 44

### Synthesis of Ru complex 35a

The synthesis procedure is the same as in Example 38.After complexation and purification, the yield for purple Ru complex solid **35a** is 68%, and the NMR results of 35a is as follow:

¹HNMR (300 MHz, CDCl₃): δ = 17.38 (d, 1H, *J* = 4.39 Hz), 8.12 (d, 1H, *J* = 2.20 Hz,), 8.01 (dd, 1H, *J* = 2.20, 8.79 Hz), 7.22 (d, 1H, *J* = 8.79 Hz), 5.35 (m, 1H), 3.79 (t, 4H, *J* = 4.77 Hz), 3.11 (t, 4H, *J* = 4.76 Hz), 2.35-1.29 (m, 39H).

### Example 45

### Synthesis of Ru complex 35b

The synthesis procedure is the same as in Example 38. After complexation and purification, the yield for purple Ru complex solid **35b** is 57%, and the NMR results of 35b is as follow:

¹HNMR (300 MHz, CDCl₃): δ = 17.38 (d, J = 4.4 Hz, 1H, Ru=CH), 8.11 (d, J = 1.8 Hz, 1H), 8.00 (dd, J = 1.8, 8.7 Hz, 1H), 7.17 (d, J = 8.7 Hz, 1H), 5.06-5.01 (m, 1H, OCH), 3.78 (t, J = 4.7 Hz, 4H), 3.11 (t, J = 4.7 Hz, 4H), 2.44-1.03 (m, 41H, PCy₃, O-ⁱBu)). ³¹P-NMR (121 MHz, CDCl₃): δ = 56.039 (s, PCy₃).

The structural formulas of prior reported representative complexes **10a-e** are as follows:

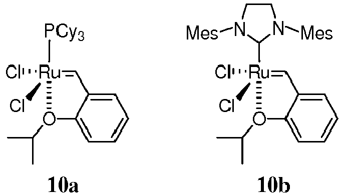

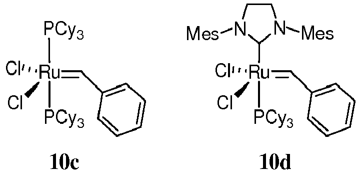

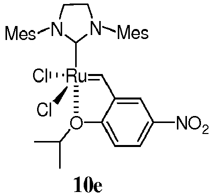

### Examples for Ru complex catalysts to catalyze olefin metathesis reactions

Experimental procedures for olefin metathesis reactions catalyzed by Ru complex catalysts: substrate (50mg) was added to a 25mL two-neck round-bottom flask filled with inleting Ar gas, freshly distilled DCM (1ml) was added to the flask. The mixture was stirred under Ar at room temperature until completed. The kinetic data for conversion was determined by HPLC. The study for the catalytic activity of Ru complexes catalyzing different olefin metathesis reactions are as follows :

### Embodiment 1:

To compare the catalytic activity of Ru complexes with different substituent groups, the olefin substrate **11** was selected to compare the relative catalytic activity of olefin metathesis reactions with different catalysts **7a-n** and **9a-9j** prepared in examples 4-26, Grubbs Ru complex catalyst **10d** substituted by ligand PCy₃ instead of isopropoxybenzylidene ligand, and Ru complex **10b**, reported by Hoveyda et al, with no substituent group in isopropoxybenzylidene ligand, respectively.

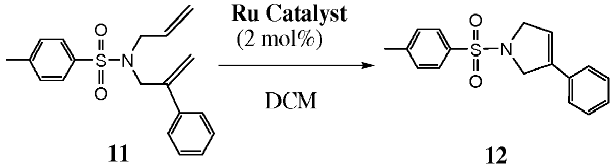

Experimental procedure for intramolecular ring-closing olefin metathesis reaction: substrate **11** (50mg) was added to a 25mL two-neck round-bottom flask filled with inleting Ar gas, freshly distilled DCM (1ml) was added to the flask by syringe needle. The mixture was stirred at room temperature until dissolved completely. After different Ru complex catalysts (2mol %) were added into each reaction, respectively. Each reaction solution was sampled at 10min, 30min, 1.0hr, 3.0hr, 5.0hr, 8.0hr and 15.0hr, respectively to determine the reaction conversion by HPLC and LC-MS. The conversion of the reaction was calculated by normalization. The results of different metathesis reactions are listed in Table 1.

The ¹HNMR results of product 12 of ring-closing olefin metathesis are as follows:

¹HNMR (400 MHz, CDCl₃): δ = 7.78 (d, 2H, J = 8.21 Hz), 7.31 (m, 7H), 6.01 (m, 1H), 4.47 (m, 2H), 4.30 (m, 2H), 2.41 (s, 3H). (M+H⁺): m/z calculated: 300.1; found: 300.2.

**Table 1. Activity Study of Substrate 11 for Intramolecular Ring-closing Reaction**

| Entry | Catalyst | Conversion (% by HPLC) | | | |
|---|---|---|---|---|---|
| | | 10 min | 30 min | 1.5 hr | 3.0 hr |
| 1 | **7a** | 85 | 96 | 100 | |
| 2 | **7b** | 88 | 100 | | |
| 3 | **7c** | 81 | 87 | 94 | >97 |
| 4 | **7d** | 83 | 91 | >97 | |
| 5 | **7e** | 51 | 82 | 92 | 100 |
| 6 | **7f** | 83 | 94 | 100 | |
| 7 | **7g** | 84 | >97 | | |
| 8 | **7h** | 87 | 98 | | |
| 9 | **7i** | 88 | >97 | | |
| 10 | **7j** | 90 | >98 | | |
| 11 | **7k** | 91 | 100 | | |
| 12 | **7m** | 89 | 94 | >98 | |
| 13 | **7n** | 80 | 91 | 94 | >97 |
| 14 | **9g** | 66 | 84 | 92 | >98 |
| 15 | **9h** | 90 | 95 | 100 | |
| 16 | **9j** | 82 | 91 | 97 | 100 |
| 17 | **10b** | 71 | 88 | 95 | >97 |
| 18 | **10d** | 12 | 23 | 37 | 81 |

Data in Table 1 show that different new catalysts of the present invention are very active for substrate **11,** and the ring-closing reactions mostly completed in 30-60 min. Complex **7k** substituted by 5-dimethylaminosulfonyl is the most active catalyst and **7j, 7b, 7i, 7h, 7i, 7m, 7f** are all very active.

### Embodiment 2:

To study the difference between several kinds of highly active catalysts, olefin substrate **13** with two electron-withdrawing chloro atoms and two methyl substituents on the olefin was designed in the present invention. Substrate **13** is much more difficult for Ru catalysts to catalyze the ring-closing metathesis reaction (RCM), so compound **13** is better to be used for evaluation of catalytic activity with highly active Ru catalysts during metathesis (RCM).

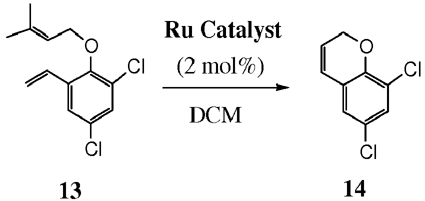

Experimental Procedure for intramolecular ring-closing olefin metathesis: multi-substituted styrene ether **13** (50mg) was added to a 25mL two-neck round-bottom flask filled with inleting Ar gas, freshly distilled DCM (1.0ml) was added to the flask by syringe needle. The mixture was stirred at room temperature until dissolved completely. After different Ru complex catalysts (2mol %) were added into each reaction, respectively. Each reaction solution was sampled at 10min, 30min, 1.0hr, 3.0hr, 5.0hr, 8.0hr and 15.0hr, respectively to determine the reaction conversion by HPLC and LC-MS. The conversion of the reaction was calculated by normalization. The kinetic data was listed in Table 2.

The ¹HNMR results of product 14 of ring-closing olefin metathesis are as follows:

¹HNMR (CDCl₃: δ = 7.26 ppm): 7.15 (d, 1H, J = 2.74 Hz), 6.84 (d, 1H, J=2.34Hz), 6.34 (dt, 1H, J=1.95, 9.78Hz), 5.86 (d, 1H, J=9.78Hz), 4.95 (m, 2H). (M+H⁺): m/z calculated: 200.99; found: 201.1.

**Table 2. Catalytic Activity Study of Substrate 13 for Intramolecular Ring-closing Reaction**

| Entry | Catalyst | Conversion (% by HPLC) | | | | |
|---|---|---|---|---|---|---|
| | | 10 min | 30 min | 1.5 hr | 3.0 hr | Overnight |
| 1 | **7a** | 26 | 51 | 76 | 86 | 100 |
| 2 | **7f** | 28 | 54 | 89 | >98 | |
| 3 | **7i** | 23 | 47 | 75 | 88 | >96 |
| 4 | **7k** | 76 | 92 | 100 | | |
| 5 | **9a** | 45 | 59 | 89 | 100 | |
| 6 | **9b** | 85 | >98 | | | |
| 7 | **9c** | 55 | 81 | 94 | 100 | |
| 8 | **9d** | 31 | 49 | 67 | 84 | 100 |
| 9 | **9e** | 48 | 82 | 94 | 100 | |
| 10 | **9f** | 20 | 43 | 71 | 86 | >97 |
| 11 | **9g** | 32 | 59 | 78 | 89 | 100 |
| 12 | **9h** | 28 | 61 | 86 | 92 | 100 |
| 13 | **9i** | 60 | 81 | 94 | >98 | |
| 14 | **9j** | 32 | 60 | 79 | 86 | >97 |
| 15 | **19a** | 2 | 5 | 23 | 46 | 100 |
| 16 | **19b** | 7 | 28 | 61 | 75 | 100 |
| 17 | **10b** | 9 | 18 | 32 | 63 | >95 |
| 18 | **10d** | 3 | 7 | 16 | 52 | 92 |
| 19 | **10e** | 49 | 77 | 89 | 100 | |

Results in the embodiment 1 and 2 show that compared with similar products such as Grubbs catalyst **10d** and Hoveyda catalyst **10b**, the catalytic activity of most Ru complex catalysts with aminosulfonyl (R₂NSO₂), carboxyl substituents etc. in the present invention is much better than other similar Ru catalysts. So far, the six catalysts **7j, 7k, 9a, 9b, 9c, 9i** are the best catalysts of all studied Ru catalysts for olefin metathesis reactions.

The ¹HNMR results of product 14 of ring-closing olefin metathesis are as follows:

¹HNMR (400 MHz, CDCl₃): δ = 7.15 (d, 1H, J = 2.74 Hz), 6.84 (d, 1H, J=2.34Hz), 6.34 (dt, 1H, J=1.95, 9.78Hz), 5.86 (d, 1H, J=9.78Hz), 4.95 (m, 2H). (M+H⁺): m/z calculated: 201.1; found: 201.1.

### Embodiment 3:

To evaluate the catalytic activity of immobilized Ru complex catalysts **19a** and **19b,** the catalytic activity of immobilized Ru complexes **19a** and **19b** synthesized in example 28 and 29 was determined by catalyzing the olefin metathesis reactions of substrate **20.**

Experimental procedure for intramolecular ring-closing olefin metathesis: substrate styrene allyl ether **20** (50mg) was added to a 25mL two-neck round-bottom flask filled with inleting Ar gas, freshly distilled DCM (1.0ml) was added to the flask by syringe needle. The mixture was stirred at room temperature until dissolved completely. After different Ru complex catalysts (2mol %) were added into each reaction, respectively. Each reaction solution was sampled at 10min, 30min, 1.0hr, 3.0hr, 5.0hr, 8.0hr and 15.0hr, respectively to determine the reaction conversion by HPLC and LC-MS. The conversion of the reaction was calculated by normalization.

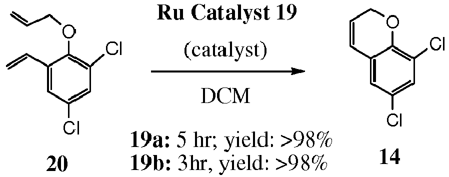

It shows that the reactions catalyzed by immobilized Ru complex catalysts of the present invention were completed in 3hr, 5hr respectively, and single product was obtained. The reaction solution is quite clear. The purity of product **14** is higher than 95% after filtration and solvent removal.

The product and its results of NMR and mass spectra are the same as in embodiment 2.

### Embodiment 4:

To evaluate the catalytic activity of immobilized Ru complex catalysts **19a** and **19b,** the catalytic activity of immobilized Ru complex catalysts **19a** and **19b** synthesized in example 28 and 29 was determined for catalyzing olefin metathesis reactions of alkyl substituted substrate **22.**

Experiment for intramolecular ring-closing olefin metathesis: substrate styrene allyl ether **20** (50mg) was added to a 25mL two-neck round-bottom flask filled with inleting Ar gas, freshly distilled DCM (1.0ml) was added to the flask by syringe needle. The mixture was stirred at room temperature until dissolved completely. After different Ru complex catalysts (2mol %) were added into each reaction, respectively. Each reaction solution was sampled at 10min, 30min, 1.0hr, 3.0hr, 5.0hr, 8.0hr and 15.0hr, respectively to determine the reaction conversion by HPLC and LC-MS. The conversion of the reaction was calculated by normalization.

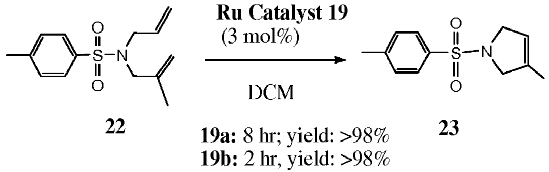

It shows that the reactions catalyzed by immobilized Ru complex catalysts of the present invention were completed in 2hr, 8hr respectively, and single products were obtained. The reaction liquid is undertint. The purity of product 23 after filtering and removal solvent can be higher than 95%. Final treatment of the reaction is easy and pure product can be obtained by filtering and removing solvent.

The NMR results of product 23 of olefin metathesis are as follows:

¹HNMR (400 MHz, CDCl₃): δ = 7.70 (d, 2H, J = 8.19 Hz), 7.31 (d, 1H, J=8.61Hz), 5.21 (d, 1H, J=1.17Hz), 4.06 (m, 2H), 3.96 (s, 2H), 2.42 (s, 3H), 1.70 (s, 3H).

(M+H⁺): m/z calculated: 238.1; found: 238.2.

### Embodiment 5:

To evaluate the catalytic activity of Ru complexes, the catalytic activity of Ru complexes **9a** and **9d** synthesized in example 17 and 20 were determined for catalyzing intramolecular olefin metathesis reactions.

Experiment for intramolecular cyclized olefin metathesis: substrate styrene **39** (50mg) was added to a 25mL two-necked flask filled with inleting Ar gas, freshly distilled DCM (1.0ml) was added to the flask by syringe needle. The mixture was stirred at room temperature until dissolved completely. After the Ru complex catalyst (2mol %) was added into each reaction, respectively. Each reaction solution was sampled at 10min, 30min, 1.0hr, 3.0hr, 5.0hr, 8.0hr and 15.0hr, respectively to determine the reaction conversion by HPLC and LC-MS. The conversion of the reaction was calculated by normalization..

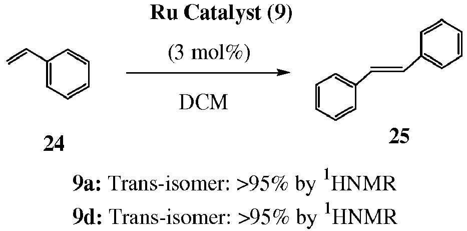

It shows that the reactions catalyzed by Ru catalysts **9a** and **9d** of the present invention were completed in 1hr respectively, and the yield of trans-isomer product **25** was higher than 95%.

The NMR results of product **25** of olefin metathesis are as follows:

¹HNMR (CDCl₃: δ = 7.26 ppm): 7.54 (d, 4H, J = 7.24 Hz), 7.39 (t, 4H, J=7.43Hz), 7.28 (t, 2H, J=7.43Hz), 7.14 (s, 2H). (M+H⁺): m/z calculated: 181.1; found: 181.2.

### Embodiment 6:

Besides the novel stable and active Ru catalysts above, intermediate complex **34a** with PCy_{3,} which was generated during synthesis of catalyst **7k,** also has catalytic activity. The embodiment compared complex **34a** with other substituted complexes **35a** and **35b** in relative catalytic activity.

Experiment for intramolecular cyclized olefin metathesis: substrate **11** (50mg) was added to a 25mL two-neck flask filled with inleting Ar gas, freshly distilled DCM (1.0ml) was added to the flask by syringe needle. The mixture was stirred at room temperature until dissolved completely. After different Ru complex catalysts (2mol %) were added into each reaction, respectively. Each reaction solution was sampled at 10min, 30min, 1.0hr, 3.0hr, 5.0hr, 8.0hr and 15.0hr, respectively to determine the reaction conversion by HPLC and LC-MS. The conversion of the reaction was calculated by normalization. The results of the reactions are listed in Table 3.

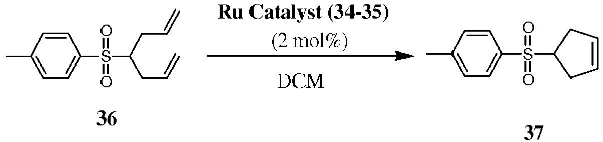

**Table 3. Activity Study of New Ru Complexes for Subsrtate 36**

| Entry | Catalyst | Conversion (% by HPLC) | | | |
|---|---|---|---|---|---|
| | | 10 min | 30 min | 1.5 hr | 3.0 hr |
| 1 | **34a** | 71 | 82 | 86 | 91 |
| 2 | **35a** | 73 | 92 | 100 | |
| 3 | **35b** | 95 | 100 | | |

The NMR results of product 37 of olefin metathesis are as follows:

¹HNMR (300 MHz, CDCl₃): δ = 7.72 (d, J = 8.2 Hz, 1H), 7.32 (d, J=8.0Hz, 1H), 5.66 (d, J=4.4 Hz, 1H), 4.11 (d, J=4.4Hz, 1H), 2.42 (s, 3H).

(M+H⁺): m/z calculated: 222.1; found: 222.2.

### Embodiment 7:

Substrate **38** with dimethyl substituted one alkenyl group was designed to compare the catalytic activity farther with Ru catalysts **34a** and **35b.**

Experiment for intramolecular cyclized olefin metathesis: substrate **11** (50mg) was added to a 25mL two-neck flask filled with inleting Ar gas, freshly distilled DCM (1.0ml) was added to the flask by syringe needle. The mixture was stirred at room temperature until dissolved completely. After different Ru complex catalysts (2mol %) were added into each reaction, respectively. Each reaction solution was sampled at 10min, 30min, 1.0hr, 3.0hr, 5.0hr, 8.0hr and 15.0hr, respectively to determine the reaction conversion by HPLC and LC-MS. The conversion of the reaction was calculated by normalization. The results of the metathesis reactions are listed in Table 4. It shows that the novel Ru complex catalyst **35b** with PCy₃ ligand has much higher catalytic activity than the other two catalysts **34a** and **35a.**

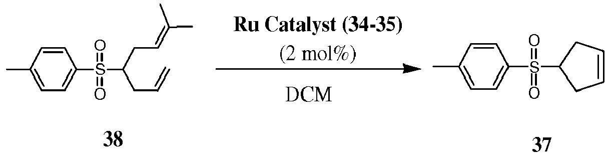

**Table 4. Catalytic Activity Study of New Ru Complexes for Subsrtate 38**

| Entry | Catalyst | Conversion (% by HPLC) | | | |
|---|---|---|---|---|---|
| | | 10 min | 30min | 1.5 hr | 3.0 hr |
| 1 | **34a** | 5 | 28 | 71 | 86 |
| 2 | **35b** | 24 | 63 | 89 | 99 |

The product in this embodiment and its results of NMR and mass spectra are the same as in embodiment 6.

The different results above show that, compared with similar Hoveyda catalyst **10b**, the catalytic activity of Ru complex catalysts **(7a-7n, 9a-9j)** with aminosulfonyl (R₂NSO₂), carboxyl substituents in the present invention is much better than other similar Ru catalysts. So far, aminosulfonyl substituted Ru catalysts **7k, 9a-d** are the best catalysts of all studied Ru catalysts for olefin metathesis reactions.

Different aminosulfonyl (R₂NSO₂), and carboxyl substituted benylidene complex ligands **(6a-6n, 8a-8j)** are first prepared and used to synthesize Ru complexes such as four Ru complexes **7k, 9a, 9b** and **9i** which are not only stable green solid but also highly active for olefin metathesis reaction as well.

Compared Ru complex catalysts in the present invention with similar products such as Grubbs catalyst **10d,** Hoveyda catalyst **10b** and Grela catalyst **10e,** the catalytic activity of most Ru complex catalysts with aminosulfonyl (R₂NSO₂) and carboxyl substituents in the present invention is much better than other similar Grubbs-Hoveyda catalysts. Among them, the six catalysts **7j, 7k, 9a, 9b, 9c, 9i** are much better in catalytic activity and the best catalysts for olefin metathesis reactions. The present invention can generate single product with the immobilized Ru complex catalysts, and purity of product **23** can be higher than 95% after filtration. Final work-up of the metathesis reaction is easy to obtain pure product by filtration and removal of solvent, and Ru complex catalysts can be recovered and reused.

### Statements of equipments and raw materials in embodiments are as follows:

Infra-red spectrometric data are analyzed by Infra-red spectrometric analyzer, Fourier Transform AVATAR™ 360 E.S.P™, Thermo Nicolet Company (unit as cm⁻¹).

¹HNMR is detected by Varian Mercury Plus 400 (400MHz) Nuclear Magnetic Resonance Spectrometer. Chemical shift is recorded by tetramethylsilane as inter label, unit as ppm(CHCl3: δ =7.26ppm). The data are as follow: chemical shift, spilting and coupling constant (s: singlet peak, d: doublet peak, t: triplet peak, q: quartet peak, br: broad peak, m: multiplet peak).

Expect for other requirement, mass spectra data are all analyzed by Finnigan LCQ Advantage Liquid Chromatograph-Mass Spectrometer. All reactions are operated in anhydrous and oxygen free condition under dried Argon gas. Solid metal organic compounds are all stored in drying cabinet under an Ar.

All silica gel (200-300 mesh) of column chromatogram are all purchased from Qingdao Ocean Chemical Plant.

Trtrahydrofuran and ethylether are distilled with sodium and diphenyl ketone. Dichloromethane, pentane and hexane are all dried by calcium hydride. Cl₂Ru=CHPh(PCy₃)(H₂IMes) is synthesized according to reference (Jason S. Kingsbury, Joseph P. A. Harrity, Peter J. Bonitatebus, Jr., Amir H. Hoveyda*, J. Am. Chem. Soc. 1999, 121, 791 ). All the other reagents are purchased from Shanghai Reagent Company.

## Claims

1. A ruthenium complex ligand having the following structure **I:** 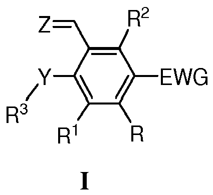 wherein:
Y is oxygen, sulfur, nitrogen or phosphorus;
Z is methylene, oxygen or p-toluenesulfonyl hydrazone;
R is hydrogen, halogen atom, nitro, cyano, C₁-C₂₀ alkyl, C₁-C₂₀ alkoxy, C₁-C₂₀ alkylthio, C₁-C₂₀ silanyl, C₁-C₂₀ silanyloxy, C₆-C₂₀ aryl, C₆-C₂₀ aryloxy, C₂-C₂₀ heterocyclic, C₂-C₂₀ heterocyclic aryl, sulfinyl, sulfonyl, formyl, C₁-C₂₀ carbonyl, C₁-C₂₀ ester, C₁-C₂₀ amido, C₁-C₂₀ uramido or derivatives, or C₁-C₂₀ sulfonamido group;
R¹ and R² are each hydrogen , bromo, I, C₁-C₂₀ alkyl or derivatives , C₁-C₂₀ alkoxy, C₁-C₂₀ alkylthio, C₁-C₂₀ silanyloxy, C₆-C₂₀ aryloxy, C₆-C₂₀ aryl, C₂-C₂₀ heterocyclic, C₂-C₂₀ heterocyclic aryl, C₁-C₂₀ ester, C₁-C₂₀ amido, C₁-C₂₀ uramido or derivatives, or C₁-C₂₀ sulfonamido group;
R³ is hydrogen, C₁-C₂₀ alkyl or derivatives , C₁-C₂₀ alkoxy, C₁-C₂₀ alkylthio, C₁-C₂₀ silanyl, C₁-C₂₀ silanyloxy, C₆-C₂₀ aryl, C₆-C₂₀ aryloxy, C₂-C₂₀ heterocyclic, C₂-C₂₀ heterocyclic aryl, sulfinyl, sulfonyl, C₁-C₂₀ carbonyl, C₁-C₂₀ ester, C₁-C₂₀ amido, C₁-C₂₀ uramido or derivatives, or C₁-C₂₀ sulfonamido group;
EWG is chloro.

2. The ruthenium complex ligand according to claim 1, wherein in structure I,
Y is oxygen or sulfur;
Z is oxygen, methylene or p-toluenesulfonyl hydrazone;
R is hydrogen, halogen atom, nitro, cyano, C₁-C₁₅ alkyl, C₁-C₁₅ alkoxy, C₁-C₁₅ alkylthio, C₁-C₁₅ silanyl, C₁-C₁₅ silanyloxy, C₆-C₁₅ aryl, C₆-C₁₅ aryloxy, C₂-C₁₅ heterocyclic, C₂-C₁₅ heterocyclic aryl, sulfinyl, sulfonyl, formyl, C₁-C₁₅ carbonyl, C₁-C₁₅ ester, C₁-C₁₅ amido, C₁-C₁₅ uramido or derivatives, or C₁-C₁₅ sulfonamido group;
R¹ and R² are each hydrogen, bromo, I, C₁-C₁₅ alkyl or derivatives , C₁-C₁₅ alkoxy, C₁-C₁₅ alkylthio, C₁-C₁₅ silanyloxy, C₆-C₁₅ aryloxy, C₆-C₁₅ aryl, C₂-C₁₅ heterocyclic, C₂-C₁₅ heterocyclic aryl, C₁-C₁₅ ester, C₁-C₁₅ amido, C₁-C₁₅ uramido or derivatives, or C₁-C₁₅ sulfonamido group;
R³ is hydrogen, C₁-C₁₅ alkyl or derivatives, C₁-C₁₅ alkoxy, C₁-C₁₅ alkylthio, C₁-C₁₅ silanyl, C₁-C₁₅ silanyloxy, C₆-C₁₅ aryl, C₆-C₁₅ aryloxy, C₂-C₁₅ heterocyclic, C₂-C₁₅ heterocyclic aryl, C₁-C₁₅ carbonyl, C₁-C₁₅ amido, C₁-C₁₅ uramido or derivatives, or C₁-C₁₅ sulfonamido group;
EWG is chloro.

3. The ruthenium complex ligand according to claim 2, wherein in structure I, Y is oxygen, Z is methylene or p-toluenesulfonyl hydrazone, R¹ and R² are each hydrogen, R³ is C₁-C₆ alkyl derivatives such as isopropyl or isobutyl ect., R is hydrogen, chloro fluoro, C₁-C₈ carbonyl, C₁-C₈ ester, C₁-C₈ aminocarbonyl, C₁-C₈ amido, C₁-C₈ uramido or derivatives, or C₁-C₈ sulfonamido group; EWG is chloro, an electron withdrawing group.

4. A ruthenium complex having the following structure **II:** 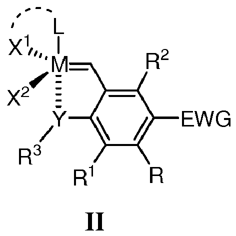 wherein,
M is ruthenium;
X¹ and X² are each chloro or R'COO, R' is C₁-C₂₀ alkyl or derivatives;
L is an electron donating complex ligand, which could be linked or not linked with X¹ forming cyclic structure.
Y, R, R¹, R², R³ and EWG each is as defined in claim 1, respectively.

5. The ruthenium complex according to claim 4, wherein L can be selected from the following structures **IIIa, IIIb, IIIc** or **IIId:** 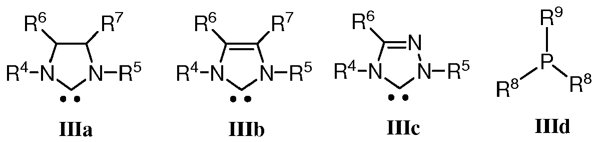 wherein:
R⁴ and R⁵ are each C₁-C₂₀ alkyl, C₆-C₂₀ aryl, C₂-C₂₀ heterocyclic aryl, C₁-C₂₀ heterocyclic, C₁-C₂₀ carbonyl, C₁-C₂₀amido, C₁-C₂₀uramido or derivatives, or C₁-C₂₀ sulfonamido group;
R⁶ and R⁷ are each hydrogen, C₁-C₂₀ alkyl, C₁-C₂₀ alkoxy, C₁-C₂₀ alkylthio, C₁-C₂₀ silanyl, C₁-C₂₀ silanyloxy, C₆-C₂₀ aryl, C₆-C₂₀ aryloxy, C₂-C₂₀ heterocyclic aryl, C₂-C₂₀ heterocyclic, sulfinyl, sulfonyl, C₁-C₂₀ carbonyl, C₁-C₂₀ ester, C₁-C₂₀ amido, C₁-C₂₀ uramido or derivatives, C₁-C₂₀ sulfonamido, halogen atom, nitro or cyano group;
R⁸ and R⁹ are each C₁-C₂₀ alkyl or derivatives, C₁-C₂₀ alkoxy, C₆-C₂₀ aryl, C₆-C₂₀ aryloxy, C₂-C₂₀ heterocyclic aryl, or C₂-C₂₀ heterocyclic group.

6. The ruthenium complex according to claim 5, wherein structural formula of L is structure **IIIa,** R⁴ and R⁵ are each 2,4,6-trimethylphenyl, R⁶ and R⁷ are each hydrogen or **IIId,** R⁸ and R⁹ are each cyclohexyl.

7. The ruthenium complex according to claim 5, wherein in structure **II,**
X¹ and X² are each chloro anion;
L is **IIIa** or **IIId;**
Y is oxygen;
R is hydrogen, halogen atom, nitro, cyano, C₁-C₁₅ alkyl, C₁-C₁₅ alkoxy, C₁-C₁₅ alkylthio, C₁-C₁₅ silanyl, C₁-C₁₅ silanyloxy, C₆-C₁₅ aryl, C₆-C₁₅ aryloxy, C₂-C₁₅ heterocyclic, C₂-C₁₅ heterocyclic aryl, sulfinyl, sulfonyl, formyl, C₁-C₁₅ carbonyl, C₁-C₁₅ ester, C₁-C₁₅ amido, C₁-C₁₅ uramido or derivatives, or C₁-C₁₅ sulfonamido group;
R¹ and R² are each hydrogen, bromo, iodo, C₁-C₁₅ alkyl or derivatives, C₁-C₁₅ alkoxy, C₁-C₁₅ alkylthio, C₁-C₁₅ silanyloxy, C₆-C₁₅ aryloxy, C₆-C₁₅ aryl, C₂-C₁₅ heterocyclic, C₂-C₁₅ heterocyclic aryl, C₁-C₁₅ ester, C₁-C₁₅ amido, C₁-C₁₅ uramido or derivatives, or C₁-C₁₅ sulfonamido group;
R³ is hydrogen, C₁-C₁₅ alkyl or derivatives, C₁-C₁₅ alkoxy, C₁-C₁₅ alkylthio, C₁-C₁₅ silanyl, C₁-C₁₅ silanyloxy, C₆-C₁₂ aryl, C₆-C₁₂ aryloxy, C₂-C₁₂ heterocyclic, C₂-C₁₂ heterocyclic aryl, C₁-C₁₂ carbonyl, C₁-C₁₂ amido, C₁-C₁₂ uramido or derivatives, or C₁-C₁₂ sulfonamido group;
EWG is chloro.

8. The ruthenium complex according to claim 7, wherein in **IIIa,** R⁴ and R⁵ are each aryl group, R⁶ and R⁷ are each hydrogen.

9. The ruthenium complex according to claim 8, wherein in the structure II, Y is oxygen, R¹ and R² are each hydrogen, R³ is isopropyl or C₁-C₆ alkyl, R is hydrogen, chloro, fluoro, C₁-C₈ carbonyl, C₁-C₈ ester, C₁-C₈ aminocarbonyl, C₁-C₈ amido, C₁-C₈ uramido or derivatives or C₁-C₈ sulfonamido group; EWG is chloro, an electron withdrawing group.

10. Use of Ru complexes according to claim 4 in olefin metathesis reactions as catalysts.

11. The use according to claim 10, wherein to carry out olefin metathesis reactions including intramolecular ring-closing olefin metathesis reactions, intermolecular olefin metathesis reactions, or olefin metathesis polymerization reactions.
